# EUROPEAN PATENT APPLICATION

(11) **EP 4 186 925 A1**
(43) Date of publication of application: **31.05.2023**
(21) Application number: 21845245.6
(22) Date of filing: 20.07.2021
(51) Int. Cl.: C07K 16/28, A61K 39/395, C12N 15/13, C12N 15/63, C12N 5/10, G01N 33/577

(54) **CD8 BINDING POLYPEPTIDE AND USE THEREOF**

(30) Priority: 21.07.2020 CN 202010703962
(71) Applicant: Suzhou Smartnuclide Biopharmaceutical Co., Ltd., Jiangsu 215100 (CN)
(72) Inventor: XU, Tao, Suzhou, Jiangsu 215100 (CN); SUN, Yan, Suzhou, Jiangsu 215100 (CN); WANG, Chao, Suzhou, Jiangsu 215100 (CN); QI, Hao, Suzhou, Jiangsu 215100 (CN); DU, Haijing, Suzhou, Jiangsu 215100 (CN); YANG, Yanling, Suzhou, Jiangsu 215100 (CN)
(74) Representative: Michalski Hüttermann & Partner Patentanwälte mbB
(86) International application number: PCT/CN2021/107312
(87) International publication number: WO 2022/017370

(57) **Abstract**

The present invention relates to the field of biological medicines. Specifically, the present invention relates to a specific CD8 binding polypeptide and use thereof.

## Description

### Technical Field

The present invention relates to the field of biological medicines. Specifically, the present invention relates to a specific CD8 binding polypeptide and use thereof.

### Background

Emission Computed Tomography (ECT) has been used for diagnosis of tumors. ECT comprises Single-photo Emission Computed Tomography (SPECT) and Positron Emission Tomography (PET), which allow high-resolution tumor imaging and quantitative analysis through images.

In human, CD8 is mainly expressed on cytotoxic T lymphocytes, but is also expressed on dendritic cells, natural killer cells and the like. CD8 molecule can be a homodimer formed by CD8α, or a heterodimer formed by CD8α and CD8β, wherein αβ heterodimer is more common.

The ability of monitoring CD8 positive tumor infiltrating lymphocytes (TILs) in vivo is of great significance for evaluating the response of immunotherapy and assisting in the development of a more effective immunocyte targeting mono- or combined therapy. "Immuno PET" imaging of tumor infiltrating lymphocytes can provide a specific and sensitive method which helps patients to select a specific immunotherapeutic regimen and determine whether the treatment is effective.

There is a need in the art for a detecting agent to detect cells expressing CD8, particularly a CD8 antibody-based detecting agent suitable for ECT detection.

### Summary of the Invention

The present invention at least comprises the following embodiments:
Embodiment 1: A CD8 binding polypeptide, comprising at least one immunoglobulin single variable domain capable of specifically binding to CD8α, the at least one immunoglobulin single variable domain comprising CDR1, CDR2 and CDR3 of any one of SEQ ID NOs: 5, 9, 13, 17, 21, 25, 29, 33, 37, 41, 45, 49, 53, 57, 61, 65, 69, 73, 77 and 81.
Embodiment 2: The CD8 binding polypeptide according to Embodiment 1, wherein the at least one immunoglobulin single variable domain comprises CDR1, CDR2 and CDR3 selected from:
   (1) CDR1 of SEQ ID NO:2, CDR2 of SEQ ID NO: 3 and CDR3 of SEQ ID NO:4;
   (2) CDR1 of SEQ ID NO:6, CDR2 of SEQ ID NO: 7 and CDR3 of SEQ ID NO:8;
   (3) CDR1 of SEQ ID NO: 10, CDR2 of SEQ ID NO: 11 and CDR3 of SEQ ID NO: 12;
   (4) CDR1 of SEQ ID NO:14, CDR2 of SEQ ID NO: 15 and CDR3 of SEQ ID NO:16;
   (5) CDR1 of SEQ ID NO:18, CDR2 of SEQ ID NO: 19 and CDR3 of SEQ ID NO:20;
   (6) CDR1 of SEQ ID NO:22, CDR2 of SEQ ID NO: 23 and CDR3 of SEQ ID NO:24;
   (7) CDR1 of SEQ ID NO:26, CDR2 of SEQ ID NO: 27 and CDR3 of SEQ ID NO:28;
   (8) CDR1 of SEQ ID NO:30, CDR2 of SEQ ID NO: 31 and CDR3 of SEQ ID NO:32;
   (9) CDR1 of SEQ ID NO:34, CDR2 of SEQ ID NO: 35 and CDR3 of SEQ ID NO:37;
   (10) CDR1 of SEQ ID NO:38, CDR2 of SEQ ID NO: 39 and CDR3 of SEQ ID NO:40;
   (11) CDR1 of SEQ ID NO:42, CDR2 of SEQ ID NO: 43 and CDR3 of SEQ ID NO:44;
   (12) CDR1 of SEQ ID NO:46, CDR2 of SEQ ID NO: 47 and CDR3 of SEQ ID NO:48;
   (13) CDR1 of SEQ ID NO:50, CDR2 of SEQ ID NO: 51 and CDR3 of SEQ ID NO:52;
   (14) CDR1 of SEQ ID NO:54, CDR2 of SEQ ID NO: 55 and CDR3 of SEQ ID NO:56;
   (15) CDR1 of SEQ ID NO:58, CDR2 of SEQ ID NO: 59 and CDR3 of SEQ ID NO:60;
   (16) CDR1 of SEQ ID NO:62, CDR2 of SEQ ID NO: 63 and CDR3 of SEQ ID NO:64;
   (17) CDR1 of SEQ ID NO:66, CDR2 of SEQ ID NO: 67 and CDR3 of SEQ ID NO:68;
   (18) CDR1 of SEQ ID NO:70, CDR2 of SEQ ID NO: 71 and CDR3 of SEQ ID NO:72;
   (19) CDR1 of SEQ ID NO:74, CDR2 of SEQ ID NO: 75 and CDR3 of SEQ ID NO:76;
   (20) CDR1 of SEQ ID NO:78, CDR2 of SEQ ID NO: 79 and CDR3 of SEQ ID NO:80.
Embodiment 3: The CD8 binding polypeptide according to Embodiment 1 or 2, wherein the immunoglobulin single variable domain comprises an amino acid sequence having at least 80%, preferably at least 90%, more preferably at least 95%, even more preferably at least 99% sequence identity to an amino acid sequence of one of SEQ ID NOs: 5, 9, 13, 17, 21, 25, 29, 33, 37, 41, 45, 49, 53, 57, 61, 65, 69, 73, 77 and 81.
Embodiment 4: The CD8 binding polypeptide according to any one of Embodiments 1-3, wherein the immunoglobulin single variable domain comprises an amino acid sequence of one of SEQ ID NOs: 5, 9, 13, 17, 21, 25, 29, 33, 37, 41, 45, 49, 53, 57, 61, 65, 69, 73, 77 and 81.
Embodiment 5: The CD8 binding polypeptide according to any one of Embodiments 1-4, wherein the immunoglobulin single variable domain is a VHH.
Embodiment 6: A nucleic acid molecule encoding the CD8 binding polypeptide according to any one of Embodiments 1-5.
Embodiment 7: An expression vector, comprising the nucleic acid molecule according to Embodiment 6 operatively linked to an expression regulatory element.
Embodiment 8: A host cell, comprising the nucleic acid molecule according to Embodiment 6 or being transformed by the expression vector according to Embodiment 7, and being capable of expressing the CD8 binding polypeptide.
Embodiment 9: A method for generating the CD8 binding polypeptide according to any one of Embodiments 1-5, comprising:
   a) culturing the host cell according to Embodiment 8 under the condition allowing the expression of the CD8 binding polypeptide;
   b) recovering the CD8 binding polypeptide expressed by the host cell from the culture obtained in step a); and
   c) optionally further purifying and/or modifying the CD8 binding polypeptide obtained in step b).
Embodiment 10: A conjugated molecule, comprising the CD8 binding polypeptide according to any one of Embodiments 1-5, and at least one detectable label conjugated to the CD8 binding polypeptide.
Embodiment 11: The conjugated molecule according to Embodiment 10, wherein the detectable label is selected from a radionuclide, a fluorescent agent, a chemiluminescent agent, a bioluminescent agent, a paramagnetic ion and an enzyme.
Embodiment 12: The conjugated molecule according to Embodiment 11, wherein the detectable label is selected from ¹¹⁰In, ¹¹¹In, ¹⁷⁷Lu, ¹⁸F, ⁵²Fe, ⁶²Cu, ⁶⁴Cu, ⁶⁷Cu, ⁶⁷Ga, ⁶⁸Ga, ⁶⁸Ge, ⁸⁶Y, ⁹⁰Y, ⁸⁹Zr, ^{94m}Tc, ¹²⁰I, ¹²³I, ¹²⁴I, ¹²⁵I, ¹³¹I, ¹⁵⁴⁻¹⁵⁸Gd, ³²P, ¹¹C, ¹³N, ¹⁵O, ¹⁸⁶Re, ¹⁸⁸Re, ⁵¹Mn, ^{52m}Mn, ⁵⁵Co, ⁷²As, ⁷⁵Br, ⁷⁶Br, ⁸²mRb, ⁸³Sr or other γ-, β- or positive emitters, for example, the detectable label is ⁶⁸Ga or ¹²⁵I.
Embodiment 13: The conjugated molecule according to any one of Embodiments 10-12, wherein the CD8 binding polypeptide is conjugated to the detectable label through a chelating agent.
Embodiment 14: The conjugated molecule according to Embodiment 13, wherein the chelating agent is selected from DTPA, EDTA, NOTA, DOTA, TRAP, TETA, NETA, CB-TE2A, Cyclen, Cyclam, Bispidine, TACN, ATSM, SarAr, AmBaSar, MAG₃, MAG₂, HYNIC, DADT, EC, NS₃, H2dedpa, HBED, DFO, PEPA or HEHA, and derivatives thereof.
Embodiment 15: The conjugated molecule according to Embodiment 14, wherein the detectable label is ⁶⁸Ga and the chelating agent is NOTA.
Embodiment 16: A method for detecting the presence and/or amount of CD8 in a biological sample, comprising:
   a) contacting the biological sample and a control sample with the CD8 binding polypeptide of the present invention or the conjugated molecule of the present invention under the condition that the CD8 binding polypeptide according to any one of Embodiments 1-5 or the conjugated molecule according to any one of Embodiments 10-15 can form a complex with CD8; and
   b) detecting the formation of the complex,
   wherein the difference in the formation of the complex between the biological sample and the control sample indicates the presence and/or amount of CD8 in the sample.
Embodiment 17: A detecting agent for detecting a CD8 positive cell, comprising the CD8 binding polypeptide according to any one of Embodiments 1-5 or the conjugated molecule according to any one of Embodiments 10-15, and optionally a physiologically acceptable carrier.
Embodiment 18: The detecting agent according to Embodiment 17, wherein the detecting agent is a contrast agent.
Embodiment 19: The detecting agent according to Embodiment 18, wherein the contrast agent is an Emission Computed Tomography (ECT) contrast agent, such as a Single Photon Emission Computed Tomography (SPECT) contrast agent or a Positron Emission Tomography (PET) contrast agent.
Embodiment 20: Use of the CD8 binding polypeptide according to any one of Embodiments 1-5 or the conjugated molecule according to any one of Embodiments 10-15 in preparation of a detecting agent for detecting a CD8 positive cell.
Embodiment 21: The detecting agent according to Embodiment 20, wherein the detecting agent is a contrast agent.
Embodiment 22: The detecting agent according to Embodiment 21, wherein the contrast agent is an ECT contrast agent, such as an SPECT contrast agent or a PET contrast agent.
Embodiment 23: A method for detecting the presence and/or amount of CD8 positive cell(s) in a tissue, comprising:
   a) contacting the tissue with the conjugated molecule according to any one of Embodiments 10-15 or the detecting agent according to any one of Embodiments 17-19; and
   b) determining the presence and/or amount of the CD8 positive cell(s) in the tissue.
Embodiment 24: The method according to Embodiment 23, wherein the tissue is selected from a blood tissue, a lymphatic tissue and a tumor tissue.
Embodiment 25: The method according to Embodiment 23 or 24, wherein the CD8 positive cell is a CD8 positive T cell.
Embodiment 26: The method according to any one of Embodiments 23-25, wherein the presence and/or amount of the CD8 positive cell(s) in the tissue is determined by imaging the tissue.
Embodiment 27: The method according to any one of Embodiments 23-25, wherein the presence and/or amount of the CD8 positive cell(s) in the tissue is determined by flow cytometry.
Embodiment 28: A method for detecting the presence and/or amount of CD8 positive cell(s) in a tissue of a subject, comprising administrating the conjugated molecule according to any one of Embodiments 10-15 or the detecting agent according to any one of Embodiments 17-19 to the subject.
Embodiment 29: The method according to embodiment 28, wherein the tissue is a tumor tissue.
Embodiment 30: The method according to Embodiment 28 or 29, wherein the CD8 positive cell is a CD8 positive T cell.
Embodiment 31: The method according to any one of Embodiments 28-30, wherein the method further comprises a step of imaging the subject, such as ECT imaging, for example the ECT imaging is SPECT imaging or PET imaging.
Embodiment 32: A method for determining whether a subject with a tumor is suitable for an anti-tumor therapy, the method comprises:
   1) administrating the conjugated molecule according to any one of Embodiments 10-15 or the detecting agent according to any one of Embodiments 17-19 to the subject, and
   2) imaging the subject, such as ECT imaging, to determine whether the tumor of the subject contains CD8 positive cell(s),
   wherein, if the presence of CD8 positive cell(s) in the tumor is detected, for example the tumor of the subject is infiltrated by CD8 positive cell(s), the subject is identified as being suitable for the anti-tumor therapy.
Embodiment 33: A method for predicting the response of a subject with a tumor to an anti-tumor therapy, the method comprises:
   1) administrating the conjugated molecule according to any one of Embodiments 10-15 or the detecting agent according to any one of Embodiments 17-19 to the subject, and
   2) imaging the subject, such as ECT imaging, to determine whether the tumor of the subject contains CD8 positive cell(s),
   wherein, if the presence of CD8 positive cell(s) in the tumor is detected, for example the tumor of the subject is infiltrated by CD8 positive cell(s), the subject may respond to the anti-tumor therapy.
Embodiment 34: A method for treating a tumor in a subject, the method comprising:
   1) administrating the conjugated molecule according to any one of Embodiments 10-15 or the detecting agent according to any one of Embodiments 17-19 to the subject, and
   2) imaging the subject, such as ECT imaging, to determine whether the tumor of the subject contains CD8 positive cell(s),
   wherein, if the presence of CD8 positive cell(s) in the tumor is detected, for example the tumor of the subject is infiltrated by CD8 positive cell(s), applying the anti-tumor therapy to the subject.
Embodiment 35: A method for monitoring the efficacy of an anti-tumor therapy in a subject, the method comprising:
   1) administrating the conjugated molecule according to any one of Embodiments 10-15 or the detecting agent according to any one of Embodiments 17-19 to a subject with a tumor and treated with the anti-tumor therapy; and
   2) imaging the subject, such as ECT imaging, to determine the amount of CD8 positive cell(s) in the tumor of the subject.
Embodiment 36: Tthe method according to any one of Embodiments 32-35, wherein the anti-tumor therapy is an immunocheckpoint inhibitor therapy.
Embodiment 37: The method according to any one of Embodiments 32-36, wherein the anti-tumor therapy is selected from administration of PD-1 inhibitors, PD-L1 inhibitors, CTLA-4 inhibitors, TIM3 inhibitors, BTLA inhibitors, TIGIT inhibitors, CD47 inhibitors, GITR inhibitors, LAG3 inhibitors, antagonists of another T cell co-inhibitor or ligand, indoleamine-2,3-dioxygenase (IDO) inhibitors, vascular endothelial growth factor (VEGF) antagonists, Ang2 inhibitors, transforming growth factor β (TGFβ) inhibitors, epidermal growth factor receptor (EGFR) inhibitors, CD20 inhibitors, antibodies for tumor-specific antigens, vaccines, adjuvants to increase antigen presentation, bispecific antibodies, cytotoxins, chemotherapeutic agents, cyclophosphamide, radiotherapy, IL-6R inhibitors, IL-4R inhibitors, IL-10 inhibitors, cytokines and antibody-drug conjugates (ADC).
Embodiment 38: The method according to any one of Embodiments 32-37, wherein the tumor is a solid tumor.
Embodiment 39: The method according to Embodiment 38, wherein the solid tumor is selected from colorectal cancer, ovarian cancer, prostate cancer, breast cancer, brain cancer, cervical cancer, bladder cancer, anal cancer, uterine cancer, colon cancer, liver cancer, pancreatic cancer, lung cancer, endometrial cancer, bone cancer, testicular cancer, skin cancer, kidney cancer, stomach cancer, esophageal cancer, head and neck cancer, salivary gland cancer, and myeloma.
Embodiment 40: A method for isolating and/or purifying CD8 positive cell(s), the method comprising:
   (a) providing a cell population suspected to contain CD8 positive cell(s);
   (b) identifying a subpopulation of the cell population, wherein the cells of the subpopulation bind to the CD8 binding polypeptide according to any one of Embodiments 1-5 or the conjugated molecule according to any one of Embodiments 10-15; and
   (c) isolating the subpopulation.
Embodiment 41: A method for isolating CD8 positive cell(s), the method comprising:
   (a) providing a cell population suspected to contain CD8 positive cell(s);
   (b) contacting the cell population with the CD8 binding polypeptide according to any one of Embodiments 1-5 or the conjugated molecule according to any one of Embodiments 10-15, thereby allowing the CD8 positive cells to bind to the CD8 binding polypeptide according to any one of Embodiments 1-5 or the conjugated molecule according to any one of Embodiments 10-15;
   (c) removing cells that do not bind to the CD8 binding polypeptide according to any one of Embodiments 1-5 or the conjugated molecule according to any one of Embodiments 10-15; and
   (d) recovering the CD8 positive cells that bind to the CD8 binding polypeptide according to any one of Embodiments 1-5 or the conjugated molecule according to any one of Embodiments 10-15.
Embodiment 42: The method according to Embodiment 40 or 41, wherein the CD8 positive cell is a CD8 positive T cell.
Embodiment 43: The method according to any one of Embodiments 40-42, wherein the cell population comprising CD8 positive cells is human peripheral blood mononuclear cell (PBMC).
Embodiment 44: The method according to any one of Embodiments 40-44, wherein the CD8 binding polypeptide according to any one of Embodiments 1-5 or the conjugated molecule according to any one of Embodiments 10-15 is immobilized onto a solid surface, for example is immobilized onto a gel or the surface of a magnetic bead.
Embodiment 45: A kit, comprising the CD8 binding polypeptide according to any one of Embodiments 1-5 or the conjugated molecule according to any one of Embodiments 10-15 or the detecting agent according to any one of Embodiments 17-19.

### Brief Description of the Drawings

FIG.1: investigation of the binding effect of candidate antibodies on human PBMC CD8+T cell surface antigen by fluorescence activated cell sorting (FACS).
FIG.2: Labeling CD8 single domain antibody with ¹²⁵I.
FIG.3: MC38-CD8 cell uptake experiment of ¹²⁵I labeled CD8α single domain antibody.
FIG.4: MC38-CD8 cell saturation binding analysis of ¹²⁵I labeled CD8 single domain antibody.
FIG.5: MC38-CD8 cell competitive binding analysis of ¹²⁵I-SNA006a.
FIG.6: SPECT imaging with ¹²⁵I labeled CD8α single domain antibody.
FIG.7: Thin-layer chromatography (TLC) analysis of ⁶⁸Ga labeled CD8α single domain antibody.
FIG.8: In-vivo distribution data of ⁶⁸Ga labeled CD8α single domain antibody.
FIG.9: In-vivo distribution target to background ratio of ⁶⁸Ga labeled CD8α single domain antibody.
FIG.10: In-vivo PET/CT imaging with ⁶⁸Ga labeled CD8α single domain antibody.
FIG.11: In-vivo time-biological distribution curve of ⁶⁸Ga labeled CD8α single domain antibody.
FIG. 12: In-vivo stability analysis of ⁶⁸Ga-NOTA-SNA006a.

### Detailed Description of the Invention

### Definition

Unless indicated or defined otherwise, all terms used have their usual meaning in the art, which will be clear to the skilled person. Reference is for example made to the standard handbooks, such as Sambrook et al, "Molecular Cloning: A Laboratory Manual" (2nd.Ed.), Vols. 1-3, Cold Spring Harbor Laboratory Press (1989); Lewin, "Genes IV", Oxford University Press, New York, (1990); and Roitt et al., "Immunology" (2nd edition), Gower Medical Publishing, London, New York (1989), and the general prior arts cited herein. In addition, unless otherwise stated, all methods, steps, techniques, and operations that are not specifically detailed can be and have been performed in a manner known per se, which will be clear to the skilled person. Reference is also for example made to the standard manual, the above-mentioned general prior arts and other references cited herein.

Unless indicated otherwise, the term "antibody" or "immunoglobulin" used interchangeably herein", whether refer to a heavy chain antibody or to a conventional 4-chain antibody, are used as general terms to include both the full-size antibody, the individual chains thereof, as well as all parts, domains or fragments thereof (including but not limited to antigen-binding domains or fragments such as VHH domains or VH/VL domains, respectively). In addition, the term "sequence" as used herein (for example in terms like "immunoglobulin sequence", "antibody sequence", "(single) variable domain sequence", "VHH sequence" or "protein sequence"), should generally be understood to include both the relevant amino acid sequence as well as nucleic acid sequences or nucleotide sequences encoding the same, unless the context requires a more limited interpretation.

The term "domain" (of a polypeptide or protein) as used herein refers to a folded protein structure which has the ability to retain its tertiary structure independently of the rest of the protein. Generally, domains are responsible for discrete functional properties of proteins, and in many cases may be added, removed or transferred to other proteins without loss of function of the remainder of the protein and/or of the domain.

The term "immunoglobulin domain" as used herein refers to a globular region of an antibody chain (such as e.g. a chain of a conventional 4-chain antibody or of a heavy chain antibody), or to a polypeptide that essentially consists of such a globular region. Immunoglobulin domains are characterized in that they retain the immunoglobulin fold characteristic of antibody molecules The term "immunoglobulin variable domain" as used herein means an immunoglobulin domain essentially consisting of four "framework regions" which are referred to in the art and herein below as "framework region 1" or "FR1"; as "framework region 2" or"FR2"; as "framework region 3" or "FR3"; and as "framework region 4" or "FR4", respectively; which framework regions are interrupted by three "complementarity determining regions" or "CDRs", which are referred to in the art and herein below as "complementarity determining region 1" or "CDR1"; as "complementarity determining region 2" or "CDR2"; and as "complementarity determining region 3" or "CDR3", respectively. Thus, the general structure or sequence of an immunoglobulin variable domain can be indicated as follows: FR1-CDR1-FR2-CDR2-FR3-CDR3-FR4. It is the immunoglobulin variable domain(s) that confer specificity to an antibody for the antigen by carrying the antigen-binding site.

The term "immunoglobulin single variable domain" as used herein means an immunoglobulin variable domain which is capable of specifically binding to an epitope of the antigen without pairing with an additional variable immunoglobulin domain. One example of immunoglobulin single variable domains in the meaning of the invention is "domain antibodies", such as the immunoglobulin single variable domains VH and VL (VH domains and VL domains). Another example of immunoglobulin single variable domains is "VHH domains" (or simply "VHHs") from camelids, as defined hereinafter.

"VHH domains", also known as VHHs, VHH domains, VHH antibody fragments, and VHH antibodies, have originally been described as the antigen binding immunoglobulin (variable) domain of "heavy chain antibodies" (i.e. "antibodies devoid of light chains") (Hamers-Casterman C, Atarhouch T, Muyldermans S, Robinson G, Hamers C, Songa E B, Bendahman N, Hamers R.: "Naturally occurring antibodies devoid of light chains"; Nature 363, 446-448 (1993)). The term "VHH domain" has been chosen in order to distinguish these variable domains from the heavy chain variable domains that are present in conventional 4-chain antibodies (which are referred to herein as "VH domains") and from the light chain variable domains that are present in conventional 4-chain antibodies (which are referred to herein as "VL domains"). VHH domains can specifically bind to an epitope without an additional antigen binding domain (as opposed to VH or VL domains in a conventional 4-chain antibody, in which case the epitope is recognized by a VL domain together with a VH domain). VHH domains are small, robust and efficient antigen recognition units formed by a single immunoglobulin domain.

In the context of the present invention, the terms "heavy chain single domain antibody", "VHH domain", "VHH", "VHH domain", "VHH antibody fragment" and "VHH antibody" can be interchangeably used.

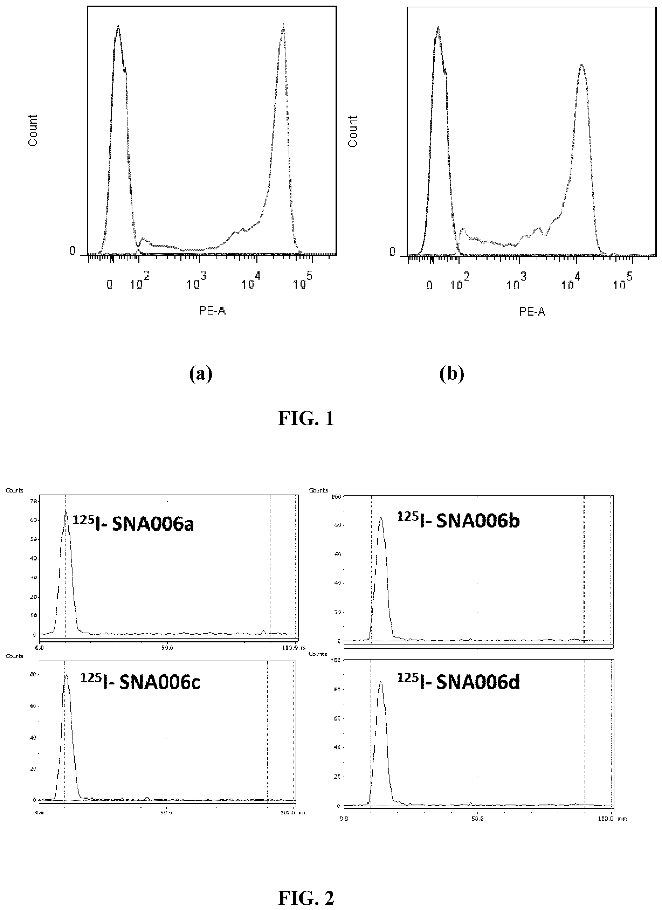

The amino acid residues of a immunoglobulin single variable domain, e.g. a VHH, are numbered according to the general numbering for VH domains given by Kabat et al. ("Sequence of Proteins of Immunological interest", 5th ED. Public Health Services, NIH Bethesda, Md., (1991)), as applied to VHH domains from Camelids, as shown e.g. in FIG. 2 of Riechmann and Muyldermans, J. Immunol. Methods 231, 25-38 (1999).

Alternative methods for numbering the amino acid residues of the VH domain are known in the art, and the alternative methods can also be similarly applied to VHH domains. For example, Chothia CDR refers to positions in structural loops (Chothia and Lesk, J. Mol. Biol. 196:901-917 (1987)). AbM CDR represents the compromise between Kabat hypervariable region and Chothia structural loop, and is used in Oxford Molecular's AbM antibody modeling software. The "Contact" CDR is based on the analysis on the crystal structure of the available complex. The residues of CDR from each method are described as follows:

| **Ring** | **Kabat** | **AbM** | **Chothia** | **Contact** |
|---|---|---|---|---|
| LCDR1 | L24-L34 | L24-L34 | L26-L32 | L30-L36 |
| LCDR2 | L50-L56 | L50-L56 | L50-L52 | L46-L55 |
| LCDR3 | L89-L97 | L89-L97 | L91-L96 | L89-L96 |
| HCDR1 (Kabat numbering) | H31-H35B | H26-H35B | H26-H32 | H30-H35B |
| HCDR1 (Chothia numbering) | H31-H35 | H26-H35 | H26-H32 | H30-H35B |
| HCDR2 | H50-H65 | H50-H58 | H53-H55 | H47-H58 |
| HCDR3 | H95-H102 | H95-H102 | H96-H101 | H93-H101 |

However, it should be noted that, as is well known in the art for VH domains and for VHH domains, the total number of amino acid residues in each of the CDRs may vary and may not correspond to the total number of amino acid residues indicated by the Kabat numbering (that is, one or more positions according to the Kabat numbering may not be occupied in the actual sequence, or the actual sequence may comprise more amino acid residues than the number enabled for by the Kabat numbering). This means that, generally, the numbering according to Kabat may or may not correspond to the actual numbering of the amino acid residues in the actual sequence.

For example, CDR can include an "extended CDR", for example 24-36 or 24-34 (LCDR1), 45-56 or 50-56 (LCDR2) and 89-97 or 89-96 (LCDR3) in VL; 26-35 (HCDR1), 50-56 or 49-65 (HCDR2) and 93-102, 94-102 or 95-102 (HCDR3) in VH.

The total number of amino acid residues in a VHH domain will usually be in the range of from 110 to 120, often between 112 and 115. It should however be noted that smaller and longer sequences may also be suitable for the purposes described herein.

Further structural characteristics and functional properties of VHH domains and polypeptides containing the same can be summarized as follows:
VHH domains (which have been "designed" by nature to functionally bind to an antigen without the presence of, and without any interaction with, a light chain variable domain) can function as a single, relatively small, functional antigen-binding structural unit, domain or polypeptide. This distinguishes the VHH domains from the VH and VL domains of conventional 4-chain antibodies, which by themselves are generally not suitable for practical application as single antigen-binding proteins or immunoglobulin single variable domains, but need to be combined in some form or another to provide a functional antigen-binding unit (as in for example conventional antibody fragments such as Fab fragments; in scFvs, which consist of a VH domain covalently linked to a VL domain).

Because of these unique properties, the use of VHH domains - either alone or as part of a larger polypeptide - offers a number of significant advantages over the use of conventional VH and VL domains, scFvs or conventional antibody fragments (such as Fab- or F(ab')2-fragments): only a single domain is required to bind an antigen with high affinity and with high selectivity, so that there is no need to have two separate domains present, nor to assure that these two domains are present in the right spacial conformation and configuration (i.e. through the use of especially designed linkers, as with scFvs); VHH domains can be expressed from a single gene and require no post- translational folding or modifications; VHH domains can easily be engineered into multivalent and multispecific formats (formated); VHH domains are highly soluble and do not have a tendency to aggregate; VHH domains are highly stable to heat, pH, proteases and other denaturing agents or conditions and, thus, may be prepared, stored or transported without the use of refrigeration equipments, conveying a cost, time and environmental savings; VHH domains are easy and relatively cheap to prepare, even on a scale required for production; VHH domains are relatively small (approximately 15 kDa, or 10 times smaller than a conventional IgG) compared to conventional 4-chain antibodies and antigen-binding fragments thereof, and therefore show high(er) penetration into tissues and can be administered in higher doses than such conventional 4-chain antibodies and antigen-binding fragments thereof; VHH domains can show so-called cavity-binding properties (especially due to their extended CDR3 loop, compared to conventional VH domains) and can therefore also access targets and epitopes not accessible to conventional 4-chain antibodies and antigen-binding fragments thereof.

Methods of obtaining VHH domains binding to a specific antigen or epitope have been described earlier, e.g. in R. van der Linden et al., Journal of Immunological Methods, 240 (2000) 185-195; Li et al., J Biol Chem., 287 (2012) 13713-13721; Deffar et al., African Journal of Biotechnology Vol. 8 (12), pp. 2645-2652, 17 June, 2009 and WO94/04678.

VHH domains derived from camelids can be "humanized" (also termed "sequence-optimized" herein, "sequence-optimizing" may, in addition to humanization, encompass an additional modification of the sequence by one or more mutations that furnish the VHH with improved properties, such as the removal of potential post translational modification sites) by replacing one or more amino acid residues in the amino acid sequence of the original VHH sequence by one or more of the amino acid residues that occur at the corresponding position(s) in a VH domain from a conventional human 4-chain antibody. A humanized VHH domain can comprise one or more fully human framework region sequences. Humanization can be accomplished by using method of humanizing protein surface amino acids (resurfacing) and/or a humanization universal framework CDR grafting method (CDR grafting to a universal framework).

As used herein, the term "epitope" or the term "antigenic determinant" used interchangeably refers to any antigenic determinant on the antigen to which the paratope of an antibody binds. Antigenic determinants usually comprise chemically active surface groups of molecules, such as amino acids or sugar side chains, and usually have specific three-dimensional structural characteristics and specific charge characteristics. For example, an epitope usually includes at least 3, 4, 5, 6, 7, 8, 9, 10, 11, 12, 13, 14, or 15 consecutive or non-consecutive amino acids in a unique spatial conformation, which can be a "linear epitope" or a "conformational" epitope. See, for example, Epitope Mapping Protocols in Methods in Molecular Biology, Vol. 66, G.E. Morris, Ed. (1996). In the linear epitope, all interaction points between a protein and an interacting molecule (such as an antibody) exist linearly along the protein's primary amino acid sequence. In a conformational epitope, interaction points exist across protein amino acid residues that are separated from each other.

Many epitope mapping techniques well known in the art can be used to identify an epitope of a given antigen. See, for example, Epitope Mapping Protocols in Methods in Molecular Biology, Volume 66, G.E. Morris, Ed. (1996). For example, linear epitopes can be determined by, for example, the following method: synthesizing a large number of peptides on a solid support at the same time, wherein these peptides correspond to parts of the protein molecule, and reacting these peptides with antibodies while still attached to the support. These techniques are known in the art and are described in, for example, U.S. Patent No. 4,708,871; Geysen et al. (1984) Proc. Natl. Acad. Sci. USA 81: 3998-4002; Geysen et al. (1986) Molec. Immunol. 23:709-715. Similarly, conformational epitopes can be identified by determining spatial configuration of amino acids, such as by, for example, x-ray crystallography and 2-dimensional nuclear magnetic resonance. See, for example, Epitope Mapping Protocols (ibid.).

Conventional techniques known to those skilled in the art can be used to screen antibodies for competitively binding to the same epitope. For example, competition and cross-competition studies can be conducted to obtain antibodies that compete with each other or cross-compete for binding to an antigen. A high-throughput method for obtaining antibodies that bind to the same epitope based on their cross-competition is described in international patent application WO03/48731. Therefore, conventional techniques known to those skilled in the art can be used to obtain antibodies and antigen-binding fragments thereof that compete with the antibody molecules of the invention for binding to the same epitope on CD8.

Generally, the term "specificity" refers to the number of different types of antigens or epitopes to which a particular antigen-binding molecule or antigen- binding protein (such as an immunoglobulin single variable domain of the invention) molecule can bind. The specificity of an antigen-binding protein can be determined based on its affinity and/or avidity. The affinity, represented by the equilibrium constant for the dissociation of an antigen with an antigen- binding protein (KD), is a measure for the binding strength between an epitope and an antigen-binding site on the antigen-binding protein: the lesser the value of the KD, the stronger the binding strength between an epitope and the antigen-binding protein (alternatively, the affinity can also be expressed as the affinity constant (KA), which is 1/KD). As will be clear to the skilled person, affinity can be determined in a manner known per se, depending on the specific antigen of interest. Avidity is the measure of the strength of binding between an antigen-binding protein (such as an immunoglobulin, an antibody, an immunoglobulin single variable domain or a polypeptide containing it) and the pertinent antigen. Avidity is related to both the affinity between an epitope and its antigen binding site on the antigen-binding molecule and the number of pertinent binding sites present on the antigen-binding protein.

As used herein, the term "CD8 binding protein (CD8 binding polypeptide)" means any proteins capable of binding a CD8α protein. The CD8 binding protein can comprise an antibody against CD8α, for example an antibody as defined herein. The CD8 binding protein also covers an immunoglobulin superfamily antibody (IgSF) or a CDR grafting molecule. Exemplary amino acid sequence of CD8α is as shown in SEQ ID NO:1.

"CD8 binding protein" of the present invention can comprise at least one immunoglobulin single variable domain, such as VHH, which binds to CD8. In some embodiments, the "CD8 binding molecule" of the present invention can comprise 2, 3, 4 or more immunoglobulin single variable domains, such as VHH, which binds to CD8. The CD8 binding protein of the present invention, except the immunoglobulin single variable domain that binds to CD8, can comprise a linker and/or a moiety having an effector function, for example a half-life period extending moiety (such as an immunoglobulin single variable domain binding serum albumin) and/or a fused ligand (such as serum albumin) and/or a conjugated polymer (such as PEG) and/or a Fc region. In some embodiments, the "CD8 binding protein" of the present invention further covers a bispecific antibody which comprises an immunoglobulin single variable domain that binds to a different antigen.

In general, the CD8 binding protein of the present invention will bind to the antigen to be bound (i.e., CD8 protein) with a dissociation constant (KD) of preferably 10⁻⁷-10⁻¹⁰ mol/L (M), more preferably 10⁻⁸-10⁻¹⁰ mol/L, even more preferably 10⁻⁹-10⁻¹⁰ mol/L or lower, and/or with an association constant (KA) of at least 10⁷ M⁻¹, preferably at least 10⁸ M⁻¹, more preferably at least 10⁹ M⁻¹, more preferably at least 10¹⁰ M⁻¹, as measured in the Biacore or KinExA or Fortibio assay. Any of KD values greater than 10⁻⁴M is generally considered to indicate non-specific binding. The specific binding of an antigen binding protein to an antigen or epitope can be determined in any suitable manner known, including, for example, a surface plasmon resonance (SPR) assay, a Scatchard assay, and/or a competitive binding assay described herein (e.g., a radioimmunoassay (RIA), an enzyme immunoassay (EIA) and a sandwich competition assay).

Amino acid residues will be indicated according to the standard three-letter or one-letter amino acid code, as generally known and agreed upon in the art. When comparing two amino acid sequences, the term "amino acid difference" refers to insertions, deletions or substitutions of the indicated number of amino acid residues at a position of the reference sequence, compared to a second sequence. In case of substitution(s), such substitution(s) will preferably be conservative amino acid substitution(s), which means that an amino acid residue is replaced with another amino acid residue of similar chemical structure and which has little or essentially no influence on the function, activity or other biological properties of the polypeptide. Such conservative amino acid substitutions are well known in the art, wherein conservative amino acid substitutions preferably are substitutions in which one amino acid within the following groups (i)-(v) is substituted by another amino acid residue within the same group: (i) small aliphatic, nonpolar or slightly polar residues: Ala, Ser, Thr, Pro and Gly; (ii) polar, negatively charged residues and their (uncharged) amides: Asp, Asn, Glu and Gln; (iii) polar, positively charged residues: His, Arg and Lys; (iv) large aliphatic, nonpolar residues: Met, Leu, Ile, Val and Cys; and (v) aromatic residues: Phe, Tyr and Trp. Particularly preferred conservative amino acid substitutions are as follows: Ala into Gly or into Ser; Arg into Lys; Asn into Gln or into His; Asp into Glu; Cys into Ser; Gln into Asn; Glu into Asp; Gly into Ala or into Pro; His into Asn or into Gln; Ile into Leu or into Val; Leu into Ile or into Val; Lys into Arg, into Gln or into Glu; Met into Leu, into Tyr or into Ile; Phe into Met, into Leu or into Tyr; Ser into Thr; Thr into Ser; Trp into Tyr; Tyr into Trp or into Phe; Val into Ile or into Leu.

"Sequence identity" between two polypeptide sequences refers to percentage of identical amino acids between the sequences. "Sequence similarity" refers to percentage of amino acids that are identical or represent substitutions of conservative amino acids. Methods for evaluating degree of sequence identity between amino acids or nucleotides are known to those skilled in the art. For example, amino acid sequence identity is usually measured using a sequence analysis software. For example, the BLAST program of the NCBI database can be used to determine the identity. For the determination of sequence identity, see, for example: Computational Molecular Biology, Lesk, AM, ed., Oxford University Press, New York, 1988; Biocomputing: Informatics and Genome Projects, Smith, DW, ed., Academic Press, New York , 1993; Computer Analysis of Sequence Data, Part I, Griffin, AM, and Griffin, HG, eds., Humana Press, New Jersey, 1994; Sequence Analysis in Molecular Biology, von Heinje, G., Academic Press, 1987 and Sequence Analysis Primer, Gribskov, M. and Devereux, J., eds., M Stockton Press, New York, 1991.

A polypeptide or nucleic acid molecule is regarded as "substantially isolated" when it has been isolated from at least one other components that are normally associated with it in its natural biological sources or media (culture medium) from which the polypeptide or nucleic acid molecule is obtained (such as another protein/polypeptide, another nucleic acid, another biological component or macromolecule or at least one contaminant, impurity or trace component), compared with the sources or/and the media (culture mediums). In particular, the polypeptide or nucleic acid molecule is regarded as "substantially ioslated" when it has been purified at least 2 times, especially at least 10 times, more especially at least 100 times and up to 1000 times or more. As determined by suitable techniques (e.g. suitable chromatographic techniques, such as polyacrylamide gel electrophoresis), the "substantially isolated" polypeptide or nucleic acid molecule is preferably substantially homogeneous.

The term "subject" as used herein means a mammal, especially a primate, especially a human.

### CD8 binding polypeptide of the present invention

The present invention provides a CD8 binding polypeptide, comprising at least one immunoglobulin single variable domain capable of specifically binding to CD8. In some embodiments, the CD8 binding polypeptide is isolated. In some embodiments, the CD8 binding polypeptide specifically binds to CD8α.

In some embodiments, the at least one immunoglobulin single variable domain comprises CDR1, CDR2 and CDR3 in VHH as shown in any one of SEQ ID NOs: 5, 9, 13, 17, 21, 25, 29, 33, 37, 41, 45, 49, 53, 57, 61, 65, 69, 73, 77 and 81. The CDRs can be Kabat CDRs, AbM CDRs, Chothia CDRs or Contact CDRs. In some embodiments, the CDRs are Kabat CDRs.

In some embodiments, the at least one immunoglobulin single variable domain comprises the CDR1, CDR2 and CDR3 selected from:
(1) CDR1 of SEQ ID NO:2, CDR2 of SEQ ID NO: 3 and CDR3 of SEQ ID NO:4;
(2) CDR1 of SEQ ID NO:6, CDR2 of SEQ ID NO: 7 and CDR3 of SEQ ID NO:8;
(3) CDR1 of SEQ ID NO: 10, CDR2 of SEQ ID NO: 11 and CDR3 of SEQ ID NO: 12;
(4) CDR1 of SEQ ID NO:14, CDR2 of SEQ ID NO: 15 and CDR3 of SEQ ID NO:16;
(5) CDR1 of SEQ ID NO:18, CDR2 of SEQ ID NO: 19 and CDR3 of SEQ ID NO:20;
(6) CDR1 of SEQ ID NO:22, CDR2 of SEQ ID NO: 23 and CDR3 of SEQ ID NO:24;
(7) CDR1 of SEQ ID NO:26, CDR2 of SEQ ID NO: 27 and CDR3 of SEQ ID NO:28;
(8) CDR1 of SEQ ID NO:30, CDR2 of SEQ ID NO: 31 and CDR3 of SEQ ID NO:32;
(9) CDR1 of SEQ ID NO:34, CDR2 of SEQ ID NO: 35 and CDR3 of SEQ ID NO:37;
(10) CDR1 of SEQ ID NO:38, CDR2 of SEQ ID NO: 39 and CDR3 of SEQ ID NO:40;
(11) CDR1 of SEQ ID NO:42, CDR2 of SEQ ID NO: 43 and CDR3 of SEQ ID NO:44;
(12) CDR1 of SEQ ID NO:46, CDR2 of SEQ ID NO: 47 and CDR3 of SEQ ID NO:48;
(13) CDR1 of SEQ ID NO:50, CDR2 of SEQ ID NO: 51 and CDR3 of SEQ ID NO:52;
(14) CDR1 of SEQ ID NO:54, CDR2 of SEQ ID NO: 55 and CDR3 of SEQ ID NO:56;
(15) CDR1 of SEQ ID NO:58, CDR2 of SEQ ID NO: 59 and CDR3 of SEQ ID NO:60;
(16) CDR1 of SEQ ID NO:62, CDR2 of SEQ ID NO: 63 and CDR3 of SEQ ID NO:64;
(17) CDR1 of SEQ ID NO:66, CDR2 of SEQ ID NO: 67 and CDR3 of SEQ ID NO:68;
(18) CDR1 of SEQ ID NO:70, CDR2 of SEQ ID NO: 71 and CDR3 of SEQ ID NO:72;
(19) CDR1 of SEQ ID NO:74, CDR2 of SEQ ID NO: 75 and CDR3 of SEQ ID NO:76;
(20) CDR1 of SEQ ID NO:78, CDR2 of SEQ ID NO: 79 and CDR3 of SEQ ID NO:80.

In some embodiments, the immunoglobulin single variable domain comprises an amino acid sequence having at least 80%, preferably at least 90%, more preferably at least 95%, even more preferably at least 99% sequence identity to an amino acid sequence of one of SEQ ID NOs: 5, 9, 13, 17, 21, 25, 29, 33, 37, 41, 45, 49, 53, 57, 61, 65, 69, 73, 77 and 81. In some embodiments, the immunoglobulin single variable domain comprises an amino acid sequence of one of SEQ ID NOs: 5, 9, 13, 17, 21, 25, 29, 33, 37, 41, 45, 49, 53, 57, 61, 65, 69, 73, 77 and 81.

In some embodiments, the immunoglobulin single variable domain is a VHH. In some embodiments, the immunoglobulin single variable domain is humanized.

### Nucleic acid, vector and host cell

In another aspect, the present invention relates to a nucleic molecule encoding the CD8 binding polypeptide of the present invention. The nucleic acid of the present invention can be RNA, DNA or cDNA. The nucleic molecule encoding the CD8 binding polypeptide of the present invention can be selected by those skilled in the art as needed or according to conventional means. In some embodiments, the nucleic molecule encoding the CD8 binding polypeptide of the present invention comprises a nucleotide sequence selected from SEQ ID NOs:82-91.

The nucleic acids of the invention may also be in the form of a vector, which may be present in the vector and/or may be part of a vector such as a plasmid, a cosmid or YAC. The vector may especially be an expression vector, i.e., a vector that provides expression of the CD8 binding polypeptide in vitro and/or in vivo (i.e., in a suitable host cell, host organism, and/or expression system). The expression vector typically comprises at least one nucleic acid of the invention operably linked to one or more suitable expression regulatory elements (e.g., promoters, enhancers, terminators, etc.). Selection of the elements and their sequences for expression in a particular host is common knowledge to those skilled in the art. Specific examples of regulatory elements and other elements useful or essential for the expression of the CD8 binding polypeptides of the invention include, such as promoters, enhancers, terminators, integration factors, selection markers, leader sequences, reporter genes.

The nucleic acids of the invention may be prepared or obtained in a known manner (for example by automated DNA synthesis and/or recombinant DNA techniques) based on information about the amino acid sequence of the polypeptides of the invention disclosed herein, and/or may be isolated from a suitable natural sources.

In another aspect, the invention involves a host cell that expresses or is capable of expressing one or more of the CD8 binding polypeptides of the invention and/or comprises a nucleic acid or vector of the invention. Preferred host cells of the invention are bacterial cells, fungal cells or mammalian cells.

Suitable bacterial cells include cells of Gram-negative bacterial strains (e.g., *Escherichia coli* strains, *Proteus* strains, and *Pseudomonas* strains) and Gram-positive bacterial strains (e.g., *Bacillus* strains, *Streptomyces* strains, *Staphylococcus* strains, and *Lactococcus* strains.

Suitable fungal cells include cells of the species of *Trichoderma, Neurospora,* and *Aspergillus;* or include cells of the species of *Saccharomyces* (e.g., *Saccharomyces cerevisiae), Schizosaccharomyces* (e.g. *Schizosaccharomyces pombe), Pichia* (e.g. *Pichia pastoris* and *Pichia methanolica)* and *Hansenula.*

Suitable mammalian cells include, for example, HEK293 cells, CHO cells, BHK cells, HeLa cells, COS cells, and the like.

However, amphibian cells, insect cells, plant cells, and any other cells in the art for expressing a heterologous protein can also be used in the invention.

The present invention further provides a method for producing the CD8 binding polypeptide of the present invention, the method comprising the following steps:
- culturing the host cell of the present invention under the condition allowing the expression of the CD8 binding polypeptide of the present invention;
- recovering the CD8 binding polypeptide expressed by the host cell from the culture; and
- optionally further purifying and/or modifying the CD8 binding polypeptide of the present invention.

The CD8 binding polypeptide of the invention may be produced in an intracellular manner (e.g., in the cytoplasm, in the periplasm, or in inclusion bodies) in cells as described above, followed by isolation from the host cells and optionally further purification; or it may be produced in an extracellular manner (for example in the medium in which the host cells are cultured), followed by isolation from the medium and optionally further purification.

Methods and reagents for recombinant production of polypeptides, such as specific suitable expression vectors, transformation or transfection methods, selection markers, methods for inducing protein expression, culture conditions, and the like, are known in the art. Similarly, protein isolation and purification techniques suitable for the methods of making the CD8 binding polypeptides of the invention are well known to those skilled in the art.

However, the CD8 binding polypeptide of the invention can also be obtained by other methods of protein production known in the art, such as chemical synthesis, including solid phase or liquid phase synthesis.

### Conjugated molecule

In another aspect, the present invention provides a conjugated molecule, comprising the CD8 binding polypeptide of the present invention, and at least one detectable label conjugated with the CD8 binding polypeptide.

The detectable label includes but is not limited to a radionuclide, a fluorescent agent, a chemiluminescent agent, a bioluminescent agent, a paramagnetic ion and an enzyme.

Fluorescent agents that can be used for conjugation include, but are not limited to, fluorescein isothiocyanate, rhodamine, phycoerythrin, phycocyanin, allophycocyanin, o-phthalaldehyde, and fluorescamine. Chemiluminescent agents that can be used for conjugation include, but not limited to luminol, isoluminol, aromatic acridinium esters, imidazoles, acridine salts and oxalates. Bioluminescent agents that can be used for conjugation include, but are not limited to, luciferin, luciferase, and jellyfish luminescence protein. Paramagnetic ions that can be used for conjugation include, but are not limited to, chromium (III), manganese (II), iron (III), iron (II), cobalt (II), nickel (II), copper (II), neodymium (III), samarium(III), ytterbium(III), gadolinium(III), vanadium(II), terbium(III), dysprosium(III), holmium(III) and erbium(III), or radiopaque materials, such as dam, diatrizoate, ethiodized oil, gallium citrate, iocarmic acid, locetamic acid, iodamide, odipamide, lodoxamic acid, Iopromide, iohexol, iopamidol, Iopanoic Acid, ioprocemic acid, iosefamic acid, ioseric acid, locarmic Acid, Iotasul, iotetric acid, iothalamic acid, iotroxic acid, ioxaglic acid, ioxotrizoic acid, ipodate, meglumine, metrizamide, metrizoic salts, dionosil, and thallous hydroxide. Enzymes that can be used for conjugation include, but are not limited to, horseradish peroxidase and the like.

Preferably, the detectable label is a radionuclide. The radionuclide that can be used for conjugation is a radionuclide with energy between 20 KeV and 4000 KeV, including but not limited to ¹¹⁰In, ¹¹¹In, ¹⁷⁷Lu, ¹⁸F, ⁵²Fe, ⁶²Cu, ⁶⁴Cu, ⁶⁷Cu, ⁶⁷Ga, ⁶⁸Ga, ⁶⁸Ge, ⁸⁶Y, ⁹⁰Y, ⁸⁹Zr, ^{94m}Tc, ¹²⁰I, ¹²³I, ¹²⁴I, ¹²⁵I, ¹³¹I, ¹⁵⁴⁻¹⁵⁸Gd, ³²P, ¹¹C, ¹³N, ¹⁵O, ¹⁸⁶Re, ¹⁸⁸Re, ⁵¹Mn, ^{52m}Mn, ⁵⁵Co, ⁷²As, ⁷⁵Br, ⁷⁶Br, ⁸²mRb, ⁸³Sr or other γ-, β- or positive emitters. In some embodiments, the detectable label is ⁶⁸Ga or ¹²⁵I.

Methods for conjugating a detectable label to a polypeptide are well-known by those skilled in the art. For example, in some embodiments, the CD8 binding polypeptide can be conjugated with the detectable label through a chelating agent.

To label the CD8 binding polypeptide of the present invention with the radionuclide such as ⁶⁸Ga, it is necessary to react the CD8 binding polypeptide of the present invention with a reagent having a long tail that is attached with a plurality of integration groups for binding to ions. Such tails can be for example polylysine, polysaccharides, or other polymers with derivatized or derivatizable chain of side groups that can bind to chelating groups, such as ethylenediaminetetraacetic acid (EDTA), diethylenetriaminepentaacetic acid (DTPA), DOTA (1,4,7,10-tetraazacyclododecane-1,4,7,10-tetraacetic acid), NOTA, TETA, NETA, porphyrin , polyamine, crown ether, bisthiosemicarbazone, polyoxime and similar groups that are known to be useful for this purpose. A chelating agent can be attached to an antibody using standard chemical methods. In some embodiments, the detectable label is conjugated to the CD8 binding polypeptide of the present invention through a chelating agent. The used chelating agent includes but is not limited to DTPA, EDTA, NOTA, DOTA, TRAP, TETA, NETA, CB-TE2A, Cyclen, Cylam, Bispidine, TACN, ATSM, SarAr, AmBaSar, MAG3, MAG2, HYNIC, DADT, EC, NS3, H2dedpa, HBED, DFO, PEPA or HEHA and derivatives thereof, etc.

In some specific embodiments, the detectable label is ⁶⁸Ga and the chelating agent is NOTA.

In another aspect, the present invention provides a method for preparing a conjugated molecule of the present invention labeled with a radionuclide such as ⁶⁸Ga, comprising 1) conjugating the CD8 binding polypeptide of the present invention with a chelating agent to generate a conjugate of the CD8 binding polypeptide and the chelating agent; and 2) contacting the product obtained in step 1) with a radionuclide such as ⁶⁸Ga, so that the CD8 binding polypeptide is labeled by the radionuclide such as ⁶⁸Ga through the chelating action of the chelating agent. In some embodiments, the chelating agent is NOTA, and in step 1), the conjugate of the CD8 binding polypeptide and NOTA is generated by reacting the CD8 binding polypeptide with p-SCN-Bn-NOTA or p-NH2-Bn-NOTA.

In another aspect, the present invention provides a method for preparing a conjugated molecule of the present invention labeled with ¹²⁵I, comprising 1) reacting the CD8 binding polypeptide of the present invention with ¹²⁵I in the presence of chloramine T; and 2) terminating the reaction by using sodium metabisulfite.

### Detection/diagnosis use

In another aspect, the present invention provides a method for detecting the presence and/or amount of CD8 in a biological sample, comprising:
a) contacting the biological sample and a control sample with the CD8 binding polypeptide of the present invention or the conjugated molecule of the present invention under the condition that the CD8 binding polypeptide of the present invention or the conjugated molecule of the present invention can form a complex with CD8; and
b) detecting the formation of the complex,
wherein, the difference in the formation of the complex between the biological sample and the control sample indicates the presence and/or amount of CD8 in the sample. In some embodiments, the biological sample is an *ex vivo* sample.

In another aspect, the present invention provides a detecting agent for detecting a CD8 positive cell, comprising the CD8 binding polypeptide of the present invention or the conjugated molecule of the present invention, and optionally a physiologically acceptable carrier. In some embodiments, the detecting agent is used for detecting the presence and/or amount of CD8 positive cells in a tissue of a subject. In some embodiments, the tissue is a tumor tissue. In some embodiments, the CD8 positive cell is a CD8 positive T cell.

The CD8 binding polypeptide of the present invention or the conjugated molecule of the present invention is especially suitable for *in-vivo* imaging, for example is suitable for Emission Computed Tomography (ECT). For example, the CD8 binding polypeptide of the present invention or the conjugated molecule of the present invention can be applied to single-photon emission computed tomography (SPECT) and positron emission tomography (PET) according to different labels. In diagnosis, ECT can provide high-resolution imaging and quantitative analysis through images. The SPECT imaging can also include SPECT/CT imaging, and the PET imaging can also include PET/CT imaging, which can provide a better imaging effect.

Therefore, in some embodiments, the detecting agent is a contrast agent. In some embodiments, the contrast agent is an ECT contrast agent, such as an SPECT contrast agent or a PET contrast agent.

In another aspect, the present invention provides use of the CD8 binding polypeptide of the present invention or the conjugated molecule of the present invention in preparation of a detecting agent for detecting a CD8 positive cell. In some embodiments, the detecting agent is used for detecting the presence and/or amount of CD8 positive cells in a tissue of a subject. In some embodiments, the tissue is a tumor tissue. In some embodiments, the CD8 positive cell is a CD8 positive T cell. In some embodiments, the detecting agent is a contrast agent. In some embodiments, the contrast agent is an ECT contrast agent, such as an SPECT contrast agent or a PET contrast agent.

In another aspect, the present invention provides a method for detecting the presence and/or amount of CD8 positive cells in a tissue, comprising:
a) contacting the tissue with the conjugated molecule of the present invention or the detecting agent of the present invention; and
b) determining the presence and/or amount of the CD8 positive cells in the tissue.

In some embodiments, the tissue is a blood tissue. In some embodiments, the tissue is a lymphatic tissue. In some embodiments, the tissue is a tumor tissue. In some embodiments, the CD8 positive cell is a CD8 positive T cell. In some embodiments, the presence and/or amount of the CD8 positive cells in the tissue is determined by imaging the tissue. In some embodiments, the presence and/or amount of the CD8 positive cells in the tissue is determined by flow cytometry.

In another aspect, the present invention provides a method for detecting a CD8 positive cell in a subject, comprising administrating the conjugated molecule of the present invention or the detection agent of the present invention to the subject. In some embodiments, the method is used for detecting the presence and/or amount of the CD8 positive cells in the tissue of the subject. In some embodiments, the tissue is the tumor tissue. In some embodiments, the CD8 positive cell is the CD8 positive T cell.

In some embodiments, the method further comprises a step of imaging the subject, such as ECT imaging. In some embodiments, the ECT imaging is SPECT imaging. In some embodiments, the ECT imaging is PET imaging. The imaging technologies and devices for SPECT or PET scanning are well-known in the art, and any such known ECT imaging technologies and devices can be used.

In another aspect, the present invention provides a method for determining whether a subject with a tumor is suitable for an anti-tumor therapy, the method comprises:
1) administrating the conjugated molecule of the invention or the detecting agent of the invention to the subject, and
2) imaging the subject, such as ECT imaging, to determine whether the tumor of the subject contains CD8 positive cells,
wherein, if the presence of the CD8 positive cells in the tumor is detected, for example the tumor of the subject is infiltrated by the CD8 positive cells, the subject is identified as being suitable for the anti-tumor therapy.

In another aspect, the present invention provides a method for predicting the response of a subject with a tumor to an anti-tumor therapy, the method comprising:
1) administrating the conjugated molecule of the invention or the detecting agent of the invention to the subject, and
2) imaging the subject, such as ECT imaging, to determine whether the tumor of the subject contains the CD8 positive cells,
wherein, if the presence of the CD8 positive cells in the tumor is detected, for example the tumor of the subject is infiltrated by the CD8 positive cells, the subject may respond to the anti-tumor therapy.

The presence of the CD8 positive cells such as CD8 positive T cells can be a prediction marker of an anti-tumor therapy response or a prognosis marker of survival. For example, tumor infiltration of baseline CD8 positive cell is a prognosis indicator of breast cancer, head/neck and ovarian cancer survival. In addition, the tumor infiltration of the CD8 positive cells detected during the anti-PD-1 therapy or anti-PDL-1 therapy is a prediction marker of therapeutic response.

In another aspect, the present invention provides a method for treating a tumor in a subject, the method comprising:
1) administrating the conjugated molecule of the present invention or the detecting agent of the present invention to the subject, and
2) imaging the subject, such as ECT imaging, to determine whether the tumor of the subject contains CD8 positive cells,
wherein, if the presence of the CD8 positive cells in the tumor is detected, for example the tumor of the subject is infiltrated by the CD8 positive cells, applying the anti-tumor therapy to the subject.

In another aspect, the present invention provides a method for monitoring the efficacy of an anti-tumor therapy in a subject, the method comprising:
1) administrating the conjugated molecule of the present invention or the detecting agent of the present invention to a subject with a tumor and treated with the anti-tumor therapy; and
2) imaging the subject, such as ECT imaging, to determine the amount of CD8 positive cells in the tumor of the subject.

In some embodiments of various aspects of the present invention, the anti-tumor therapy is an immunocheckpoint inhibitor therapy. In some embodiments, the anti-tumor therapy comprises administration of PD-1 inhibitors (for example REGN2810, BGB-A317, Nivolumab, Pirizumab and Pamuzumab), PD-L1 inhibitors (for example Atzuzumab, Avelumab and Duvalimab), CTLA-4 inhibitors (for example Ipilimumab), TIM3 inhibitors, BTLA inhibitors, TIGIT inhibitors, CD47 inhibitors, GITR inhibitors, LAG3 inhibitors, antagonists of another T cell co-inhibitors or ligand (for example antibodies for CD-28, 2B4, LY108, LAIR1, ICOS, CD160 or VISTA), indoleamine-2,3-dioxygenase (IDO) inhibitors, vascular endothelial growth factor (VEGF) antagonists, Ang2 inhibitors (Neva monoclonal antibody), transforming growth factor β (TGFβ) inhibitors, epidermal growth factor receptor (EGFR) inhibitors (for example Erotinib and cetuximab), CD20 inhibitors (for example an anti-CD20 antibody, such as rituximab), antibodies for tumor-specific antigens (for example CA9, CA125, melanoma associated antigen 3 (MAGE3), carcinoembryonic antigen (CEA), vimentin, tumor-M2-PK, prostate specific antigen (PSA), mucin-1, MART-1 and CA19-9), vaccines (for example BCG vaccine, cancer vaccine), adjuvants to increase antigen presentation (for example granulocyte-macrophage colony stimulating factor), bispecific antibodies (for example CD3 × CD20 bispecific antibody or PSMA × CD3 bispecific antibody), cytotoxics, chemotherapeutic agents (for example Dacarbazine, temozolomide, cyclophosphamide, docetaxel, donomycin, cisplatin, carboplatin, gemcitabine, methotrexate, mitoxantrone, oxaliplatin, paclitaxel and vincristine), cyclophosphamide, radiotherapy, IL-6R inhibitors (for example Sarelumab), IL-4R inhibitors (for example Dupirumab), IL-10 inhibitors, cytokines (for example IL-2, IL-7, IL-21 and IL-15) and antibody-drug conjugates (ADC) (for example anti-CD19-DM4 ADC and anti-DS6-DM4 ADC).

In some embodiments of various aspects of the present invention, the tumor is a solid tumor, including but not limited to colorectal cancer, ovarian cancer, prostate cancer, breast cancer, brain cancer, cervical cancer, bladder cancer, anal cancer, uterine cancer, colon cancer, liver cancer, pancreatic cancer, lung cancer, endometrial cancer, bone cancer, testicular cancer, skin cancer, kidney cancer, stomach cancer, esophageal cancer, head and neck cancer, salivary gland cancer, and myeloma.

### Isolation and/or purification of CD8 positive cells

In another aspect, the present invention further provides a method for isolating and/or purifying CD8 positive cells, the method comprising:
(a) providing a cell population suspected to contain CD8 positive cells;
(b) identifying a subpopulation of the cell population, wherein the cells of the subpopulation bind to the CD8 binding polypeptide of the present invention or the conjugated molecule of the present invention; and
(c) isolating the subpopulation.

In some embodiments, the CD8 positive cells are CD8 positive T cells. In some embodiments, the cell population comprising the CD8 positive cells is a human peripheral blood mononuclear cell (PBMC).

In some embodiments, the CD8 positive cells can be isolated through flow cytometry.

In another aspect, the present invention further provides a method for isolating CD8 positive cells, the method comprising:
(a) providing a cell population suspected to contain CD8 positive cells;
(b) contacting the cell population with the CD8 binding polypeptide of the present invention or the conjugated molecule of the present invention, thereby allowing the CD8 positive cells to bind to the CD8 binding polypeptide of the present invention or the conjugated molecule of the present invention;
(c) removing cells that do not bind to the CD8 binding polypeptide of the present invention or the conjugated molecule of the present invention; and
(d) recovering the CD8 positive cells that bind to the CD8 binding polypeptide of the present invention or the conjugated molecule of the present invention.

In some embodiments, the CD8 positive cell is a CD8 positive T cell. In some embodiments, the cell population comprising the CD8 positive cells is a human peripheral blood mononuclear cell (PBMC).

In some embodiments, the CD8 binding polypeptide of the present invention or the conjugated molecule of the present invention is immobilized onto a solid surface, for example is immobilized onto a gel or surface of a magnetic bead.

### Kit

In another aspect, the present invention provides a kit, comprising the CD8 binding polypeptide of the present invention or the conjugated molecule of the present invention or the detection agent of the present invention. The kit is used for implementing the method of the present invention. The kit generally comprises a tag indicating the expected use of contents in the kit. The term "tag" comprises any written or recorded materials that are on the kit or are provided together with the kit or are provided along with the kit in other manners.

### Examples

Hereinafter, the invention will be further described by way of examples, but the invention is not limited to the scope of the described examples.

### Example 1 Screening for heavy chain single domain antibody against CD8α

The amino acid sequence of CD8α-Fc fusion protein is:

MALPVTALLLPLALLLHAARPSQFRVSPLDRTWNLGETVELKCQVLLSNPTSGCSWL
FQPRGAAASPTFLLYLSQNKPKAAEGLDTQRFSGKRLGDTFVLTLSDFRRENEGYYFCSAL
SNSIMYFSHFVPVFLPAKPTTTPAPRPPTPAPTIASQPLSLRPEACRPAAGGAGIEGRMDPKS

SDKTHTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISKA
KGQPREPQVYTLPPSRDELTKNQVSLTCLVKGFYPSDIAVEWESNGQPENNYKTTPPVLDS
DGSFFLYSKLTVDKSRWQQGNVFSCSVMHEALHNHYTQKSLSLSPGK (SEQ ID NO:1)

Amino acids 1-21 are signal peptide of CD8α receptor, amino acids 22-136 are immunoglobulin (Ig) domain of the CD8α receptor, amino acids 137-181 are Ig domain of the CD8α receptor, amino acids 182-209 are transmembrane domain of the CD8α receptor, and amino acids 210-235 are cytoplasm domain of the CD8α receptor.

In the figure, the single-line marking area is an Fc area, and the double-line marking area is a linker.

### 1.1 Construction of library

The CD8α-Fc fusion protein for immunization was expressed by CHO cells, and purified by Protein A affinity chromatography. An alpaca was selected to be immunized. After immunization, lymphocytes from alpaca peripheral blood were extracted and RNA was extracted therefrom according to the instructions of Trizol, and the concentration of RNA was measured with Nanodrop. The extracted RNA was reversely transcribed into cDNA by using SuperScript^{®} III First-Strand Synthesis System for RT-PCR kit according to the instructions. The nucleic acid fragment encoding the variable region of the heavy chain antibody was amplified through nested PCR.

The VHH fragment was purified by utilizing a DNA product purification kit, and a vector and a fragment were digested with restriction endonuclease sifi at 50°C overnight. The digested fragment was cut and recovered, and then cloned into vector pComb3XSS for phage display. The product was then electrically transformed into Escherichia coli electroreceptor cell TG1 to construct heavy chain single domain antibody phage display library against CD8α and the library was verified. Through gradient dilution and paving, the volume of the library was calculated as 5.04 × 10⁹. To detect the insertion rate of the library, 48 clones were selected at random for identification. The results showed that the insertion rate had reached 100% and the size is correct.

### 1.2 Panning for heavy chain single domain antibody against CD8α

The plate was coated with 10 µg/well CD8α-cHis fusion protein and placed at 4°C overnight. On the next day, the plate was blocked for 2 hours with 3% BSA at 37°C, washed 5 times with PBST (containing 0.05% Tween 20 in PBS), and 100 µL of phage (1.71 × 10¹¹ cfu, derived from the camel heavy chain single domain antibody phage display library constructed in 1.1) was added and acted for 1 hour at 37°C. Then, the plate was washed 10 times with PBST (0.05% Tween 20 in PBS), and then washed 10 times with a PBS solution to wash out the unbound phages. After that, 100 µL of Gly-Hcl (PH=2.5) was added into each well to act for 6-8 min at 37°C, the phages specifically binding to CD8α were dissociated and transferred to a sterile centrifuge tube, and a 1/10 volume of Tris-Hcl (PH=9.0) solution was quickly added into the sterile centrifuge tube to neutralize the buffer. 10 µL of solution after neutralization was taken for gradient dilution, the titer was measured and the panning recovery rate was calculated, and the phages after neutralization were taken to infect Escherichia coil TG1 growing in logarithmic stage to produce and purify the phages for the next screening. The same screening process was repeated for several rounds, and panning conditions were changed for each round of panning. Hence, the positive clones were enriched so as to achieve the purpose of screening the CD8-specific antibodies in the antibody library by using a phage display technology. The conditions for affinity elutriation are shown in Table below.

**Table 1 Conditions for affinity panning**

| Rounds | Antigen coating concentration (µg/mL) | Blocking solution | Added amount of library (cfu) | PBST concentration | PBST washing times |
|---|---|---|---|---|---|
| 1 | 10 | 3% BSA | 1.71×10¹¹ | 0.5% | 10 |
| 2 | 5 | 3% BSA | 2.1×10¹¹ | 0.5% | 15 |
| 3 | 1 | 3% BSA | 2.1×10¹¹ | 0.5% | 20 |

### 1.3 Screening of specific single positive clones using enzyme-linked immunosorbent assay (ELISA) of phages

After 3-4 rounds of panning, 96 clones were randomly picked from the plate subjected to the last round of panning for identification. 96 single colonies were randomly picked and respectively cultured to produce and purify phages. The plate was coated with CD8α-Fc fusion protein at 4°C overnight, then blocked with 3% BSA, and reacted for 1 hour at 37°C. Subsequently, the phage supernatant obtained after infection was added into each well (the control group was blank phage), and incubated for 1 hour at 37°C. After PBST washing, horseradish peroxidase labeled anti-M13 secondary antibody (purchased from Beijing Yiqiao Shenzhou Biotechnology Co., Ltd.) was added, and reacted for 1 hour at room temperature. After washing, a tetramethylbenzidine (TMB) chromogenic solution was added and an absorption value at 450 nm was read. Meanwhile, the plate was coated with an Fc protein at 4°C overnight, blocked with 3% BSA, and reacted for 1 hour at 37°C. After washing, the phage supernatant obtained after infection (the control group was blank phage) was added to react for 1 hour at 37°C. After washing, the horseradish peroxide labeled anti-M13 secondary antibody (purchased from Beijing Yiqiao Shenzhou Biotechnology Co., Ltd.) was added to react for 1 hour at room temperature. After that, a TMB chromogenic solution was added and an absorption value at 450 nm was read. Where, when a ratio obtained by an OD value being divided by an OD value of blank control was >=4, it was judged that a candidate antibody could bind the CD8α-Fc protein; meanwhile, a ratio obtained by the CD8α-Fc binding OD value of the above antibody capable of binding the CD8 α-Fc antigen protein being divided by the Fc protein binding OD value was >=5, it was considered that the candidate antibody could specifically bind the CD8α part rather than the Fc part. The results show that some of the antibodies screened can specifically bind CD8α rather than Fc. The bacteria in candidate clone well were transferred to an LB liquid culture medium containing 100 µg/mL ampicillin so as to perform plasmid extraction and sequencing.

**Table 2 phage ELISA identification of CD8α-Fc tag antigen**

| No. | OD450 | No. | OD450 | No. | OD450 | No. | OD450 | No. | OD450 | No. | OD450 |
|---|---|---|---|---|---|---|---|---|---|---|---|
| 1 | 2.981 | 9 | 3.013 | 17 | 3.127 | 25 | 3.127 | 33 | 3.149 | 41 | 2.951 |
| 2 | **3.208** | 10 | 3.160 | 18 | 2.962 | 26 | 1.777 | 34 | 3.096 | 42 | **3.183** |
| 3 | 2.944 | 11 | 2.957 | 19 | 3.015 | 27 | **2.738** | 35 | 3.12 | 43 | 0.557 |
| 4 | 3.092 | 12 | 3.162 | 20 | **3.188** | 28 | 2.780 | 36 | 3.162 | 44 | 2.468 |
| 5 | 3.152 | 13 | 3.249 | 21 | 3.198 | 29 | **1.746** | 37 | **3.198** | 45 | 2.420 |
| 6 | 3.079 | 14 | 3.119 | 22 | 2.506 | 30 | **2.029** | 38 | 1.534 | 46 | **2.355** |
| 7 | 3.067 | 15 | 2.572 | 23 | 3.000 | 31 | 3.067 | 39 | 3.067 | 47 | 2.507 |
| 8 | 3.230 | 16 | 1.855 | 24 | **2.353** | 32 | 2.132 | 40 | 3.406 | BLank | 0.067 |
| | | | | | | | | | | | |

| No. | OD450 | No. | OD450 | No. | OD450 | No. | OD450 | No. | OD450 | No. | OD450 |
|---|---|---|---|---|---|---|---|---|---|---|---|
| 49 | 0.152 | 57 | 0.785 | 65 | 1.830 | 73 | 0.055 | 81 | 1.645 | 89 | 1.833 |
| 50 | 0.212 | 58 | 1.501 | 66 | **1.722** | 74 | 0.359 | 82 | 0.193 | 90 | 1.774 |
| 51 | 0.088 | 59 | 1.702 | 67 | 1.426 | 75 | 1.891 | 83 | 0.051 | 91 | 1.934 |
| 52 | 0.681 | 60 | 0.069 | 68 | 0.080 | 76 | 0.193 | 84 | **0.566** | 92 | 1.691 |
| 53 | **1.195** | 61 | **1.713** | 69 | 0.129 | 77 | **1.630** | 85 | 1.351 | 93 | 1.771 |
| 54 | **1.305** | 62 | 0.256 | 70 | **1.726** | 78 | 0.110 | 86 | 1.806 | 94 | 0.167 |
| 55 | **1.609** | 63 | 1.166 | 71 | **1.618** | 79 | 1.724 | 87 | 1.330 | 95 | 1.726 |
| 56 | 1.843 | 64 | 1.831 | 72 | 1.505 | 80 | **1.438** | 88 | 1.821 | BLank | 0.067 |

**Table 3 phage ELISA identification of Fc tag antigen**

| No. | OD450 | No. | OD450 | No. | OD450 | No. | OD450 | No. | OD450 | No. | OD450 |
|---|---|---|---|---|---|---|---|---|---|---|---|
| 1 | 0.129 | 9 | 0.140 | 17 | 0.116 | 25 | 3.146 | 33 | 3.080 | 41 | 0.123 |
| 2 | **0.149** | 10 | 3.459 | 18 | 0.089 | 26 | 0.104 | 34 | 0.109 | 42 | **0.111** |
| 3 | 0.111 | 11 | 0.102 | 19 | 2.974 | 27 | **0.084** | 35 | 2.989 | 43 | 0.127 |
| 4 | 0.100 | 12 | 0.114 | 20 | **0.133** | 28 | 0.100 | 36 | 0.221 | 44 | 0.102 |
| 5 | 3.173 | 13 | 3.224 | 21 | 0.106 | 29 | **0.115** | 37 | **0.110** | 45 | 0.115 |
| 6 | 0.162 | 14 | 0.098 | 22 | 0.088 | 30 | **0.103** | 38 | 0.086 | 46 | **0.081** |
| 7 | 3.023 | 15 | 0.071 | 23 | 0.074 | 31 | 0.257 | 39 | 0.088 | 47 | 0.159 |
| 8 | 0.104 | 16 | 0.09 | 24 | **0.083** | 32 | 0.085 | 40 | 3.102 | BLank | 0.093 |
| | | | | | | | | | | | |

| No. | OD450 | No. | OD450 | No. | OD450 | No. | OD450 | No. | OD450 | No. | OD450 |
|---|---|---|---|---|---|---|---|---|---|---|---|
| 49 | 1.940 | 57 | 0.063 | 65 | 0.076 | 73 | 0.078 | 81 | 0.076 | 89 | 0.176 |
| 50 | 0.059 | 58 | 0.055 | 66 | **0.081** | 74 | 0.060 | 82 | 0.061 | 90 | 0.070 |
| 51 | 0.066 | 59 | 0.151 | 67 | 0.058 | 75 | 0.061 | 83 | 0.066 | 91 | 0.314 |
| 52 | 0.071 | 60 | 0.056 | 68 | 0.053 | 76 | 0.136 | 84 | **0.059** | 92 | 0.169 |
| 53 | **0.068** | 61 | **0.094** | 69 | 0.060 | 77 | **0.080** | 85 | 0.064 | 93 | 0.081 |
| 54 | **0.058** | 62 | 0.070 | 70 | **0.059** | 78 | 0.057 | 86 | 0.057 | 94 | 2.022 |
| 55 | **0.059** | 63 | 0.063 | 71 | **0.093** | 79 | 0.063 | 87 | 0.060 | 95 | 0.056 |
| 56 | 0.062 | 64 | 0.058 | 72 | 0.064 | 80 | **0.061** | 88 | 0.117 | BLank | 0.068 |

The protein sequence of each clone was analyzed according to sequence alignment software DNAMAN. Clones with the same CDR1, CDR2, and CDR3 sequences were all considered as a same antibody, while clones with different CDR sequences were considered as different antibodies. Finally, 20 candidate sequences were obtained:
NO.2: C2, NO.20: C20, NO.24: C24, NO.27: C27, NO.29: C29, NO.30: C30, NO.37: C37, NO.42: C42, NO.45: C45, NO.46: C46, NO.53: S5, NO.54: S6, NO.55: S7, NO.61: S13, NO.66: S18, NO.70: S22, NO.71: S23, NO.77: S29, NO.32: S80, NO.36: S84.

**Table 4 Amino acid sequences of 20 candidate single domain antibodies**

| Antibody | Sequence | | SEQ ID NO: |
|---|---|---|---|
| C2 | CDR1 | GFTLDYYAIG | 2 |
| | CDR2 | CIRIYDGNTYSAESVKG | 3 |
| | CDR3 | GSYYSCSVYPDYDMDY | 4 |
| | Full-length variable region | QVQLVESGGGLVQPGGSLRLSCAASGFTLDYYAIGWF RQAPGKEREGISCIRIYDGNTYSAESVKGRFTISSDNAK NQVYLQINTLKPEDTAVYYCAAGSYYSCSVYPDYDM DYWGKGTLVTVSS | 5 |
| C20 | CDR1 | GSIFSINTMG | 6 |
| | CDR2 | TMHSTGSTNYADSVKG | 7 |
| | CDR3 | DDTGWGTVDS | 8 |
| | Full-length variable region | QVQLVESGGGLVQPGGSLRLSCAASGSIFSINTMGWYR QAPGKQRELVATMHSTGSTNYADSVKGRFTISRDNAK KTVYLQMNSLEPDDTAVYYCHVDDTGWGTVDSWGQ GTQVTVS | 9 |
| C24 | CDR1 | GPIFSINTMG | 10 |
| | CDR2 | TMHSTGSTNYADSVKG | 11 |
| | CDR3 | DDTGWGTVDS | 12 |
| | Full-length variable region | QVQLVESGGGLVQPGGSLRLSCAASGPIFSINTMGWYR QAPGKQRELVATMHSTGSTNYADSVKGRFTISRDNAK KTVYLQMNSLKPDDTAVYYCHVDDTGWGTVDSWGQ GIQVTVSS | 13 |
| C27 | CDR1 | GSIFAINTMG | 14 |
| | CDR2 | TMHSGGSTNYADSVKG | 15 |
| | CDR3 | DDSGWGTVDS | 16 |
| | Full-length variable region | QLQLVESGGGLVQPGGSLRLSCVVSGSIFAINTMGWYR QAPGKQRELVATMHSGGSTNYADSVKGRFTISRDAAP KTVYLQMNSLKPADTAVYYCHADDSGWGTVDSWGQ GIQVTVSS | 17 |
| C29 | CDR1 | GFTSDDYVIG | 18 |
| | CDR2 | CISSSDGTTNYADSVKG | 19 |
| | CDR3 | DPMRVCPYSGTYYGILGFGTS | 20 |
| | Full-length variable region | QVQLVESGGGLVQAGGSLRLSCVASGFTSDDYVIGWF RQAPGKEREGVSCISSSDGTTNYADSVKGRFTISSDNA KNTVYLQMNSLKPEDTAVYYCAADPMRVCPYSGTYY GILGFGTSGQGTQVTVSS | 21 |
| C30 | CDR1 | GSIFAINTMG | 22 |
| | CDR2 | DMHSTGSTNYADSVKG | 23 |
| | CDR3 | DDRGWGTVDS | 24 |
| | Full-length variable region | QVQLVESGGGLVQPGGSLRLSCVASGSIFAINTMGWYR QAPGKQRELVADMHSTGSTNYADSVKGRFTISRDNAK KKVYLQMNSLKPDDTAVYYCHVDDRGWGTVDSWGQ GTQVTVSS | 25 |
| C37 | CDR1 | GLTFEDYAIG | 26 |
| | CDR2 | CIRIYDGNTYSAESVKG | 27 |
| | CDR3 | GSYYSCSVYPDYDMDY | 28 |
| | Full-length variable region | QLQLVESGGGLVQAGGSLRLSCAASGLTFEDYAIGWF RQAPGKEREGISCIRIYDGNTYSAESVKGRFTISSDNAK NQVYLQINTLKPEDTAVYYCAAGSYYSCSVYPDYDM DYWGKGTLVTVSS | 29 |
| C42 | CDR1 | GLTFEDYAIG | 30 |
| | CDR2 | CIRIYDGNTYSAESVKG | 31 |
| | CDR3 | GSYYSCSVYPDYDMDY | 32 |
| | Full-length variable region | QVQLVESGGGLVQAGGSLRLSCAASGLTFEDYAIGWF RQAPGKEREGISCIRIYDGNTYSAESVKGRFTISSDNAK NQVYLQINTLKPEDTAVYYCAAGSYYSCSVYPDYDM DYWGKGTLVTVSS | 33 |
| C45 | CDR1 | GPIFSINTMG | 34 |
| | CDR2 | TMHSTGSTNYADSVKG | 35 |
| | CDR3 | DDTGWGTVDS | 36 |
| | Full-length variable region | QVQLVESGGGLVQPGGSLRLSCAASGPIFSINTMGWYR QVPGKQRELVATMHSTGSTNYADSVKGRFTISRDNAK KTVYLQMNSLKPDDTAVYYCHVDDTGWGTVDSWGQ GTQVTVSS | 37 |
| C46 | CDR1 | GVTFDDLAIG | 38 |
| | CDR2 | TIGTSDGTAYYADSVKG | 39 |
| | CDR3 | DLDYGLGSLLPMGYDY | 40 |
| | Full-length variable region | QVQLVESGGGLVQAGGSLRLSCAASGVTFDDLAIGWF RQVPGKGREGVSTIGTSDGTAYYADSVKGRFTISSDNA KNTVYLQMNSLKPEDTAVYYCAVDLDYGLGSLLPMG | 41 |
| | | YDYWGOGTOVTVSS | |
| S5 | CDR1 | GGTFSSTAMG | 42 |
| | CDR2 | AISWSGGYTYYADSVRG | 43 |
| | CDR3 | DISGLDTVAAGIFTEPNIDY | 44 |
| | Full-length variable region | QVQLVESGGGLVQAGGSLRVSCAASGGTFSSTAMGWF RQAPGKEREFVAAISWSGGYTYYADSVRGRFTISRDN ALNTVSLQMNSLKPEDTAVYYCAADISGLDTVAAGIFT EPNIDYWGKGTLVTVSS | 45 |
| S6 | CDR1 | GTIFSINTMG | 46 |
| | CDR2 | TITSSGSTKYADSVKG | 47 |
| | CDR3 | NDVGWGTVNA | 48 |
| | Full-length variable region | QVQLVESGGGLVQPGGSLRLSCAVSGTIFSINTMGWYR QVPGKQRELVATITSSGSTKYADSVKGRFTVSRDNAKR TVFLQMDSLKPEDTAVYYCHANDVGWGTVNAWGQG TLVTVSS | 49 |
| S7 | CDR1 | GSIFSINGMG | 50 |
| | CDR2 | VITSGGKIDYLDSVKG | 51 |
| | CDR3 | YGDVRRGEVRW | 52 |
| | Full-length variable region | QLQLVESGGGLVQPGGSLRLSCAASGSIFSINGMGWYR QAPGKQREAVAVITSGGKIDYLDSVKGRFTISRDSAKN TVALQMNSLKPEDTAVYYCNAYGDVRRGEVRWGQGT QVTVSS | 53 |
| S13 | CDR1 | GSIFAINTMG | 54 |
| | CDR2 | TMHSYGSTNYADSVKG | 55 |
| | CDR3 | DDTGWGTVDS | 56 |
| | Full-length variable region | QLQLVESGGGLVQPGGSLRLSCVVSGSIFAINTMGWYR QAPGKQRELVATMHSYGSTNYADSVKGRFTISRDNAK KTVYLQMNSLKPADTAVYYCHADDTGWGTVDSWGQ GTQVTVSS | 57 |
| S18 | CDR1 | GSIFSINTMG | 58 |
| | CDR2 | TMTSHGSTNYADSVKG | 59 |
| | CDR3 | NDMGWGTVDA | 60 |
| | Full-length variable region | QLQLVESGGGLVQPGGSLRLSCAASGSIFSINTMGWYR QAPGKQRELVATMTSHGSTNYADSVKGRFTISRDNSK NTVYLQMNSLKLQDTAVYYCNANDMGWGTVDAWG QGTLVTVSS | 61 |
| S22 | CDR1 | GSIFSINTMG | 62 |
| | CDR2 | TMHSTGSTNYADSVKG | 63 |
| | CDR3 | DDMGWGTVDS | 64 |
| | Full-length variable region | QLQLVESGGGLVQPGGSLRLSCAASGSIFSINTMGWYR QAPGKQRELVATMHSTGSTNYADSVKGRFTISRDNAK KTVYLQMNSLKPDDTAVYYCHVDDMGWGTVDSWG QGTQVTVSS | 65 |
| S23 | CDR1 | GSIFSINGMG | 66 |
| | CDR2 | AITSGGKTDYLDSVKG | 67 |
| | CDR3 | YGDVQRGEVRW | 68 |
| | Full-length variable region | QVQLVESGGGLVQPGGSLRLSCAASGSIFSINGMGWY RQAPGKQRELVAAITSGGKTDYLDSVKGRFTISRDSAK NTVALQMNSLKPEDTAVYYCNAYGDVQRGEVRWGQ GTQVTVSS | 69 |
| S29 | CDR1 | GIIFSINTMG | 70 |
| | CDR2 | DISSFGSTNYADSVKG | 71 |
| | CDR3 | HDKGWGTVDS | 72 |
| | Full-length variable region | QLQLVESGGGLVQPGGSLRLSCTASGIIFSINTMGWYR QAPGKQRELVADISSFGSTNYADSVKGRFTISRDNAKN SVYLQMNSLKPEDTAVYYCNAHDKGWGTVDSWGQG TQVTVSS | 73 |
| S32 | CDR1 | GSIFSINTMG | 74 |
| | CDR2 | TMHSTGSTNYADSVKG | 75 |
| | CDR3 | DDMGWGTVDS | 76 |
| | Full-length variable region | QLQLVESGGGLVQPGGSLRLSCAASGSIFSINTMGWYR QAPGKQRELVATMHSTGSTNYADSVKGRFTISRDNAK KTVYLQMNSLKPDDTAVYYCHVDDMGWGTVDSWG QGTQVTVSS | 77 |
| S36 | CDR1 | SIRTFSDYAMA | 78 |
| | CDR2 | AISWNGVTTFYADSVKG | 79 |
| | CDR3 | GLNVGWGTHEREYDD | 80 |
| | Full-length variable region | QVQLVESGGGLVQAGGSLRLSCAASIRTFSDYAMAWF RQGPRKEREFVAAISWNGVTTFYADSVKGRFTISRDDA KNSVYLQMNSLKPEDTAVYYCAGGLNVGWGTHEREY DDWGQGTQVTVSS | 81 |

| | | | |
|---|---|---|---|
| Note: The underlined part is the CDR region of the antibody sequence. | | | |

### Example 2 Preliminary evaluation and identification of heavy chain single domain antibody against CD8α

### 2.1 Expression and purification of heavy chain single domain antibody in host bacterial Escherichia coli

The coding sequence of the candidate single domain antibody obtained by sequencing analysis in example 1 was sub-cloned into expression vector PET22b, and a recombinant plasmid with correct sequencing identification was transformed into expression host strain BL21, which was plated on the plate of LB solid medium containing 100 µg/ml ampicillin at 37°C overnight. Single colonies were selected for inoculation and culture overnight. On the next day, the overnight strains were transferred and amplified. The strains were subjected to shaker culture at 37°C until the OD value reached about 0.6, then induced by 0.5 mM IPTG and subjected to shaker culture at 28°C for overnight. On the next day, the strains were centrifuged and collected and ultrasonically broken to obtain a crude antibody extract. Then, the antibody protein was purified by nickel ion affinity chromatography. Finally, antibody proteins with a purity of more than 90% were obtained.

### 2.2 Preparation of CD8α antibody protein using mammalian cells

Primers were designed for the nucleotide sequences of the screened CD8α single domain antibodies, and the nucleotide sequence (comprising histidine label) of each antibody was amplified by PCR based on the above prokaryotic expression plasmid as a template, and then cloned into a pCDNA4 (Invitrogen, Cat V86220) vector. By gene sequencing, the correctness of the obtained target cloned gene sequences were determined.

The nucleotide sequence of C2-cHis antibody (SEQ ID NO:82):

AAGCTTGCCGCCACCATGGAGACAGACACACTCCTGCTATGGGTACTGCTGCTCT
GGGTTCCAGGTTCCACCGGTCAGGTGCAGCTCGTGGAGTCTGGGGGAGGCTTGGTGC
AGCCTGGGGGGTCTCTGAGACTCTCCTGTGCAGCCTCTGGATTCACTTTGGATTATTAT
GCCATAGGCTGGTTCCGCCAGGCCCCAGGGAAGGAGCGTGAGGGGATCTCATGTATTA
GGATTTACGATGGTAACACATACTCTGCAGAGTCCGTGAAGGGCCGATTCACCATCTCC
AGTGACAACGCCAAGAACCAGGTGTATCTGCAAATCAACACCCTGAAACCGGAGGAC
ACGGCCGTTTATTACTGTGCAGCAGGGTCCTATTACTCCTGTTCAGTTTACCCTGACTAC
GACATGGACTACTGGGGCAAAGGGACCCTGGTCACCGTCTCTTCAGGCAGCATGGATC
CTGGAGGATCTCATCATCACCACCACCATCATCACTAAGAATTC

The nucleotide sequence of C24-cHis antibody (SEQ ID NO:83):

AAGCTTGCCGCCACCATGGAGACAGACACACTCCTGCTATGGGTACTGCTGCTCT
GGGTTCCAGGTTCCACCGGTCAGGTGCAGCTCGTGGAGTCTGGGGGAGGCTTGGTGC
AGCCTGGGGGGTCTCTGAGACTCTCCTGTGCAGCCTCTGGACCCATCTTCAGTATCAAT
ACCATGGGCTGGTACCGCCAGGCTCCAGGGAAGCAGCGCGAGTTGGTCGCAACTATG
CACAGTACTGGTAGCACAAACTATGCAGACTCCGTGAAGGGCCGATTCACCATCTCCA
GAGACAACGCCAAGAAGACGGTGTACCTGCAAATGAACAGCCTGAAACCTGACGACA
CGGCCGTCTATTACTGTCATGTAGATGATACGGGTTGGGGAACCGTCGATTCCTGGGGC
CAGGGGATCCAGGTCACCGTCTCCTCGGGCAGCATGGATCCTGGAGGATCTCATCATC
ACCACCACCATCATCACTAAGAATTC

The nucleotide sequence of C27-cHis antibody (SEQ ID NO: 84:

AAGCTTGCCGCCACCATGGAGACAGACACACTCCTGCTATGGGTACTGCTGCTCT
GGGTTCCAGGTTCCACCGGTCAGTTGCAGCTCGTGGAGTCTGGGGGAGGCTTGGTGC
AGCCTGGGGGGTCTCTAAGACTCTCCTGTGTAGTGTCTGGAAGCATCTTCGCTATCAAT
ACCATGGGCTGGTACCGCCAGGCTCCAGGGAAGCAGCGCGAGTTGGTCGCAACTATG
CATAGTGGTGGTTCCACAAACTATGCAGACTCCGTGAAAGGCCGATTCACCATCTCCA
GAGACGCCGCCCCGAAAACGGTGTATCTGCAAATGAACAGCCTGAAACCTGCAGACA
CGGCCGTCTATTACTGTCATGCAGACGATTCGGGTTGGGGAACCGTCGATTCGTGGGG
CCAGGGGATCCAGGTCACCGTCTCCTCGGGCAGCATGGATCCTGGAGGATCTCATCAT
CACCACCACCATCATCACTAAGAATTC

The nucleotide sequence of C29-cHis antibody (SEQ ID NO:85):

AAGCTTGCCGCCACCATGGAGACAGACACACTCCTGCTATGGGTACTGCTGCTCT
GGGTTCCAGGTTCCACCGGTCAGGTGCAGCTCGTGGAGTCTGGGGGAGGCTTGGTGC
AGGCTGGGGGGTCTCTGAGACTCTCCTGTGTAGCCTCTGGATTCACTTCGGATGATTAT
GTCATAGGCTGGTTCCGCCAGGCCCCAGGGAAGGAGCGTGAGGGGGTCTCATGTATTA
GTAGTAGTGATGGTACCACTAACTATGCAGACTCCGTGAAGGGCCGATTCACCATCTCC
AGTGACAACGCCAAGAACACGGTGTATCTTCAAATGAACAGCCTGAAACCTGAGGAC
ACGGCCGTTTATTACTGTGCAGCAGATCCAATGCGCGTTTGCCCTTATAGTGGTACTTAC
TATGGTATCCTGGGGTTTGGTACCTCTGGCCAGGGGACCCAGGTCACCGTCTCCTCAGG
CAGCATGGATCCTGGAGGATCTCATCATCACCACCACCATCATCACTAAGAATTC

The nucleotide sequence of C37-cHis antibody (SEQ ID NO:86):

AAGCTTGCCGCCACCATGGAGACAGACACACTCCTGCTATGGGTACTGCTGCTCT
GGGTTCCAGGTTCCACCGGTCAGTTGCAGCTCGTGGAGTCTGGGGGAGGCTTGGTGC

AGGCTGGGGGGTCCCTGAGACTCTCCTGTGCAGCCTCTGGACTCACTTTCGAGGATTA
CGCCATAGGCTGGTTCCGCCAGGCCCCAGGGAAGGAGCGTGAGGGGATCTCATGTATT
AGGATTTACGATGGTAACACATACTCTGCAGAGTCCGTGAAGGGCCGATTCACCATCTC
CAGTGACAACGCCAAGAACCAGGTGTATCTGCAAATCAACACCCTGAAACCGGAGGA
CACGGCCGTTTATTACTGTGCAGCAGGGTCCTATTACTCCTGTTCAGTTTACCCTGACTA
CGACATGGACTACTGGGGCAAAGGGACCCTGGTCACCGTCTCCTCAGGCAGCATGGAT
CCTGGAGGATCTCATCATCACCACCACCATCATCACTAAGAATTC

The nucleotide sequence of C42-cHis antibody (SEQ ID NO:87):

AAGCTTGCCGCCACCATGGAGACAGACACACTCCTGCTATGGGTACTGCTCT
GGGTTCCAGGTTCCACCGGTCAGGTGCAGCTCGTGGAGTCTGGGGGAGGCTTGGTGC
AGGCTGGGGGGTCCCTGAGACTCTCCTGTGCAGCCTCTGGACTCACTTTCGAGGATTA
CGCCATAGGCTGGTTCCGCCAGGCCCCAGGGAAGGAGCGTGAGGGGATCTCATGTATT
AGGATTTACGATGGTAACACATACTCTGCAGAGTCCGTGAAGGGCCGATTCACCATCTC
CAGTGACAACGCCAAGAACCAGGTGTATCTGCAAATCAACACCCTGAAACCGGAGGA
CACGGCCGTTTATTACTGTGCAGCAGGGTCCTATTACTCCTGTTCAGTTTACCCTGACTA
CGACATGGACTACTGGGGCAAAGGGACCCTGGTCACCGTCTCTTCAGGCAGCATGGAT
CCTGGAGGATCTCATCATCACCACCACCATCATCACTAAGAATTC

The nucleotide sequence of C46-cHis antibody (SEQ ID NO:88):

AAGCTTGCCGCCACCATGGAGACAGACACACTCCTGCTATGGGTACTGCTGCTCT
GGGTTCCAGGTTCCACCGGTCAGGTGCAGCTCGTGGAGTCTGGGGGAGGCTTGGTGC
AGGCTGGGGGGTCTCTGAGACTCTCCTGTGCAGCCTCTGGAGTCACTTTCGATGATTTA
GCCATAGGCTGGTTCCGCCAGGTCCCAGGGAAGGGGCGTGAGGGGGTCTCGACCATT
GGTACTAGTGATGGTACGGCATACTATGCAGACTCCGTGAAGGGCCGATTCACCATCTC
CAGTGACAACGCCAAGAATACGGTGTATCTGCAAATGAACAGCCTGAAACCTGAGGA
CACGGCCGTTTATTACTGTGCAGTGGACCTAGACTACGGGTTGGGGTCCCTCCTCCCGA
TGGGGTATGACTACTGGGGCCAGGGGACCCAGGTCACCGTCTCCTCAGGCAGCATGGA
TCCTGGAGGATCTCATCATCACCACCACCATCATCACTAAGAATTC

The nucleotide sequence of S5-cHis antibody (SEQ ID NO:89):

AAGCTTGCCGCCACCATGGAGACAGACACACTCCTGCTATGGGTACTGCTGCTCT
GGGTTCCAGGTTCCACCGGTCAGGTGCAGCTCGTGGAGTCTGGGGGAGGATTGGTGC
AGGCTGGGGGCTCTCTGAGAGTCTCCTGTGCAGCCTCTGGAGGCACCTTCAGTAGTAC
TGCCATGGGCTGGTTCCGCCAGGCTCCAGGGAAGGAGCGTGAGTTTGTAGCAGCTATT
AGCTGGAGTGGTGGCTACACATACTATGCAGACTCCGTGAGGGGCCGATTCACCATCT
CCAGAGACAACGCCCTGAACACGGTGTCTCTGCAAATGAACAGCCTGAAACCTGAGG
ACACGGCCGTTTATTACTGTGCAGCAGATATCTCGGGGCTTGATACGGTTGCAGCTGGT
ATCTTTACGGAACCGAACATAGACTACTGGGGCAAAGGGACCCTGGTCACCGTCTCCT
CAGGCAGCATGGATCCTGGAGGATCTCATCATCACCACCACCATCATCACTAAGAATTC

The nucleotide sequence of S13-cHis antibody (SEQ ID NO:90):

AAGCTTGCCGCCACCATGGAGACAGACACACTCCTGCTATGGGTACTGCTGCTCT
GGGTTCCAGGTTCCACCGGTCAGTTGCAGCTCGTGGAGTCTGGGGGAGGCTTGGTGC
AGCCTGGGGGGTCTCTGAGACTCTCCTGTGTAGTGTCTGGAAGCATCTTCGCTATCAAT
ACCATGGGCTGGTACCGCCAGGCTCCAGGGAAGCAGCGCGAGTTGGTCGCAACTATG
CATAGTTATGGTAGCACAAACTATGCAGACTCCGTGAAGGGCCGATTCACCATCTCCAG
AGACAACGCCAAGAAAACGGTGTATCTGCAAATGAACAGCCTGAAACCTGCAGACAC

GGCCGTCTATTACTGTCATGCAGACGATACGGGTTGGGGAACCGTCGATTCCTGGGGCC
AGGGGACCCAGGTCACCGTCTCCTCGGGCAGCATGGATCCTGGAGGATCTCATCATCA
CCACCACCATCATCACTAAGAATTC

The nucleotide sequence of S36-cHis antibody (SEQ ID NO:91):

AAGCTTGCCGCCACCATGGAGACAGACACACTCCTGCTATGGGTACTGCTGCTCT
GGGTTCCAGGTTCCACCGGTCAGGTGCAGCTGGTGGAGTCTGGGGGAGGATTGGTGC
AGGCTGGGGGCTCTCTGAGACTCTCCTGTGCGGCCTCTATACGCACCTTCAGCGACTAT
GCCATGGCCTGGTTCCGCCAGGGTCCAAGGAAGGAGCGTGAGTTTGTGGCAGCGATTA
GCTGGAATGGTGTTACAACATTCTATGCAGACTCCGTGAAGGGCCGATTCACCATCTCC
AGAGACGACGCCAAGAACTCGGTGTATCTGCAAATGAACAGCCTGAAACCTGAGGAC
ACGGCCGTTTATTACTGTGCAGGAGGCCTAAATGTGGGTTGGGGTACCCACGAAAGGG
AATATGACGACTGGGGCCAGGGGACCCAGGTCACCGTCTCCTCGGGCAGCATGGATCC
TGGAGGATCTCATCATCACCACCACCATCATCACTAAGAATTC

The above single domain antibody plasmids with histidine tags constructed by recombination were transfected into HEK293 cells for antibody expression. The recombinant expression plasmids were diluted with a Freestyle293 culture medium and added with a Polyethylenimine (PEI) solution required for transformation. The plasmid/PEI mixture was respectively added into a HEK293 cell suspension, and cultured under the conditions of 37°C, 10% CO₂ and 90 rpm. After culture for 5-6 days, the transient expression culture supernatant was collected, and the histidine labeled single domain antibody fusion protein was purified by Ni⁺ resin gel affinity chromatography.

### 2.3 ELISA activity detection of different anti-CD8α single domain antibodies on human CD8α protein

The CD8 α-Fc protein was transiently expressed by HEK293 and purified by protein A affinity chromatography. The plate was coated with the obtained CD8α-Fc protein in a concentration of 0.5 µg/well at 4°C overnight, and then the gradient dilution series of the obtained anti-CD8α single domain antibody protein was added to react for 1 hour at room temperature. After washing, anti-his horseradish peroxidase labeled antibody was added to react for 1 hour at room temperature. After washing, a chromogenic solution was added, and an absorption value at 450 nm was read. The data processing and plotting analysis were performed by using software SotfMaxPro v5.4. Through four-parameter fitting, a CD8α binding curve of antibody and EC50 values (EC50 values of all tested antibodies were between 0.989 ng/mL and 4.11 ng/mL) were obtained. Of the 10 selected antibodies (see Table 5 for details), the relative activities of different anti-CD8α single domain antibodies were compared by using C2-cHis as control, so as to reflect the affinity of the antibodies on CD8α. Of the 10 tested antibodies, C37, C42, C46, S5 and S36 have relatively high binding activity.

**Table 5 ELISA results of 10 antibodies on CD8α binding**

| Name | EC50 (ng/mL) | Relative activity (%) |
|---|---|---|
| C2 | 2.199 | 100 |
| C24 | 4.588 | 47.9 |
| C27 | 2.280 | 96.4 |
| C29 | 3.552 | 61.9 |
| C2 | 3.18 | 100 |
| C37 | 0.989 | 322 |
| C42 | 1.18 | 269 |
| C46 | 1.82 | 175 |
| C2 | 4.11 | 100 |
| S5 | 2.28 | 180 |
| S13 | 2.89 | 142 |
| S36 | 1.42 | 289 |

### Example 3 In-vitro activity analysis of CD8α single domain antibody

### 3.1 Investigation on binding effect of anti-CD8α single domain antibody to cell surface CD8α through FACS

Mouse colon cancer MC38 cell lines stable transfected with human CD8α full-length protein gene were constructed to obtain MC38 cells (MC38-CD8α cells) stably expressing human CD8α proteins on the cell membrane. Prior to use, the cells were cultured to >90% convergence. The anti-CD8α single domain antibody was used to perform flow cytometry on these cells for quantitative analysis of indirect immunofluorescence staining so as to determine the quantity of receptors on surface of each cell.

The cells were collected and suspended to a concentration of 2.5 ×10⁶ cells/mL. 200 µL of cell aliquot samples were mixed with 20 µL of primary antibody, and the obtained mixture was incubated for 30 min on ice. The cells were centrifuged, washed and re-suspended, then 5 µL of secondary antibody-PE conjugate was added, and then the above mixture was incubated for 30 minutes on ice. The cells were washed twice, re-suspended and underwent flow cytometry on BD FACSCelestaTM. For each tube, at least 5×10⁴ events were collected. All the analyses were single colors, and PE was detected in FL1. Forward scattering (FS) and side scattering (SS) data proved that all the cell populations were closely clustered.

The flow cytometry was used for evaluating the in-vitro CD8α expression of cells (Table 6). The positive rates of 20 single domain antibodies on MC38-CD8α cells binding are all above 93.6%. Of the 20 single domains, the positive rates of six single domain antibodies C2, C37, C42, C46, S5 and S36 are all higher than 99.5%, and the positive rate of positive control huOKT8 (huOKT8 is an expressed and purified protein whose sequence refers to US20160024209A1) is 93.0%.

**Table 6 Binding effect of antibody on cell surface CD8α investigated by FACS**

| Name | Positive rate (%) |
|---|---|
| C2 | 99.9 |
| C20 | 98.9 |
| C24 | 99.4 |
| C27 | 93.6 |
| C29 | 98.4 |
| C30 | 98.8 |
| C37 | 99.8 |
| C42 | 99.9 |
| C45 | 99.1 |
| C46 | 99.5 |
| S5 | 99.7 |
| S6 | 97.5 |
| S7 | 99.3 |
| S13 | 96.3 |
| S18 | 99.2 |
| S22 | 97.0 |
| S23 | 99.3 |
| S29 | 99.0 |
| S32 | 99.1 |
| S36 | 99.7 |
| OKT8 | 93.0 |

### 3.2 Binding of anti-CD8α single domain antibody on human PBMC CD8 + T cell surface antigen

Human peripheral blood was isolated by magnetic beads to obtain human CD8+T cells, and the binding of anti-CD8α single domain antibody C37-his to CD8+T cells was analyzed by using anti-CD8α PE conjugate (R&D Systems, Cat # FAB1509P) as positive control.

The concentration of the cells obtained by isolation was adjusted to 2.5 × 10⁶ cells/mL. 150 µL of cell aliquot samples were mixed with 3 µg of primary antibody, and then the obtained mixture was incubated for 30 minutes on ice. After the cells were washed and re-suspended, 5 µL of secondary anti-PE conjugate was added and the above mixture was on ice in dark for 30 minutes on ice in dark. The cells were washed twice, re-suspended and underwent flow cytometry on BD FACSCelestaTM. For each tube, at least 5 × 10⁴ events were collected. All the analyses were single colors, and PE was detected in FL1. Forward scattering (FS) and side scattering (SS) data prove that all the cell populations are closely clustered.

The flow cytometry was used for evaluating the CD8α expression of T cells (FIG.1). The CD8α single domain antibody C37-chis has good binding ability to a human PBMC CD8+ T cell surface antigen.

### 3.3 Identification of binding ability of anti-CD8α single domain antibody to CD8α (ForteBio method)

The anti-CD8α single domain antibody-biontin was fixed on an SA biological sensor. Then, CD8α-chis with a concentration of 6.25-100 nM bound to a nano antibody and then dissociated. The binding dynamic variables of four antibodies C37, C42, C46 and S5 were evaluated and determined by using Octet Data Analysis version 9.0, including Kon, Koff and Kd.

The measured binding affinity of the anti-CD8α antibody is shown in Table 7. The results show that the C37-his protein has significant affinity to a CD8α target protein, its relatively high Ka value and relatively low Kd value indicate that this antibody fusion protein can quickly bind a CD8α antigen and the CD8α antigen is difficultly dissociated.

**Table 7 Binding affinity of antibodies to CD8α**

| Antibodies | K_{D}(M) | Kₒₙ(1/Ms) | K_{off}(1/s) |
|---|---|---|---|
| C37 | 6.441×10⁻¹⁰ | 4.827×10⁵ | 3.109×10⁻⁴ |
| C42 | 7.272×10⁻¹⁰ | 5.782×10⁵ | 4.205×10⁻⁴ |
| C46 | 1.081×10⁻⁹ | 2.51×10⁵ | 2.714×10⁻⁴ |
| S5 | 2.867×10⁻⁹ | 1.551×10⁵ | 4.446×10⁻⁴ |

### Example 4 In-vitro analysis of ¹²⁵I labeled CD8α single domain antibody

### 4.1 Labeling CD8α single domain antibody ¹²⁵I

For the convenience of description, the single domain antibody C37-cHis was named SNA006a, the single domain antibody C42-cHis was named SNA006b, the single domain antibody C46-cHis was named SNA006c, and the single domain antibody S5-cHis was named SNA006d. The ¹²⁵I labeling experiment was conducted by using a chloramine T method.

A 10 mg/mL chloramine T solution and a sodium metabisulfite solution were prepared by using 0.05 mol/L, pH=7.5 PBS as a solvent. 2 µL of protein was diluted to 50 µL of PB solution, 50 µL of solution was added with 1.5 mCi (10µL) Na¹²⁵I to be mixed, the chloramine T solution was added into the mixed solution to be quickly and evenly mixed, and the above materials reacted for 5 min at room temperature. 50 µL of sodium metabisulfite solution was added into the above solution obtained after reaction and mixed, and the reaction was terminated. The labeling rate was identified through a Radio-TLC scanner. The unreacted ¹²⁵I was removed via PD-10 column and meanwhile the product buffer system was changed into normal saline. The labeling results are seen in FIG.2.

### 4.2 Cell binding assay of ¹²⁵I labeled CD8α single domain antibody on MC38-CD8 cells

MC38-CD8 cells (CD8 positive) and MC38 cells (negative control) were plated in four 6-well plates with 1×10⁶ cells per well and 3 mL culture medium/well, and cultured overnight at 37°C under 5% CO₂; the cells were taken out and incubated for 30 min at 4°C; the 6-well plates were all taken out, each experiment group was divided into total binding groups and non-specific binding groups, three parallel samples for each group, so one 6-well plate corresponded to 1 group of experiment. 26 µM of culture medium that was not labeled with proteins was added into a 3-well culture medium to serve as a non-specific adsorbed control sample. The culture medium was completely changed into a corresponding (¹²⁵I labeled SNA006a, b, c and d) culture medium containing 1 µci (about 5 nM) of ¹²⁵I labeled CD8 single domain antibody; the cells were incubated for 1 h at 37°C under 5% CO₂; after incubation, the supernatant was collected, the cells were washed with pre-cooled PBS containing 1% BSA and combined into the supernatant; after the rest cell precipitates were re-suspended with PBS containing SDS, the radioactivity count cpm values of the supernatant and the precipitated cells were respectively measured on a γ counter, and decay correction was conducted.

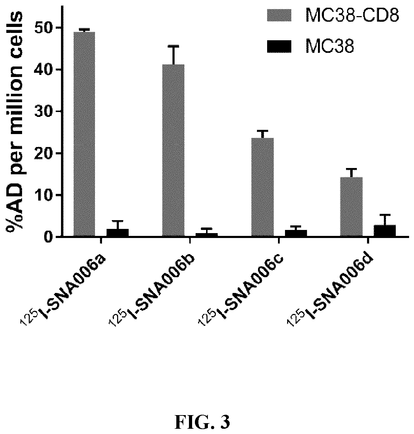

The precipitation count is the cell binding part (B) of the binding group, and the sum of the radioactivity counts of the supernatant and the precipitates is total radioactivity (T). The BIT of this group minus the corresponding non-specific binding is a radioimmunity activity fraction (calculated by %) bound by 10⁶ cells in this group, as shown in FIG.3.

As shown in FIG.3, the ¹²⁵I labeled CD8α single domain antibody has a high binding percentage to MC38-CD8 cells, while the corresponding CD8 negative cells cannot bind; furthermore, the binding of the ¹²⁵I labeled CD8α single domain antibody to MC38-CD8 cells all can be blocked by the unlabeled CD8 single domain antibody; it is proved that the specific binding ability of the ¹²⁵I labeled CD8 single domain antibody to the MC38-CD8 cells.

Meanwhile, by calculation, the binding percentage of ¹²⁵I-SNA006a on 10⁶ MC38-CD8 cells is about 50%, and the lowest binding percentage of ¹²⁵I-SNA006d is also greater than 10%. It is proved that ¹²⁵I labeled SNA006a, b, c and d all have high binding, and SNA006-a and b has a higher affinity to CD8 compared with SNA006-c and d.

### 4.3 Saturation binding assay of ¹²⁵I labeled CD8α single domain antibody

Cell preparation: a saturation test was carried out in a 96-well filter plate. MC38-CD8 cells were cultured in a 50 mL culture dish. Before the test, the culture solution was removed, the cells were washed twice with 0.01 M sterile PBS, digested with trypsin and washed twice with PBS again, and finally prepared into a cell suspension with 5% BSA. The cells were added into the 96-well suction filter plate in a concentration of about 10⁵ cells/well.

Preparation of a cold antibody solution for blocking specific binding: a 10 mg/mL CD8 single domain antibody solution (dissolved into 0.01M PBS) was diluted 10 times with 5% BSA to form a 1 mg/mL solution.

Receptor binding reaction: a radioligand saturation method was used, total binding and non-specific binding groups were included. 12 dose points with gradually increased concentrations were set for total binding groups, 12 dose points were set for non-specific binding groups, 4 groups of parallel samples were set for each dose point, the total reaction volume was 200µL, and correspondingly the cold antibody was1000 fold excess.

The cells were incubated for 2 h at 4°C. After that, the supernatant was extracted, the cells were washed 6 times with PBS pre-cooled at 4°C, and 200 µL of PBS was added into each well every time. Finally, the filter membrane was collected, the radioactivity count of each tube of sample and standard sample was measured on the γ counter. Finally, saturation curve fitting was performed by using Prism 7.0 (GraphPad Software, Inc.) software according to the single-point binding model, and then KD and Bmax were calculated.

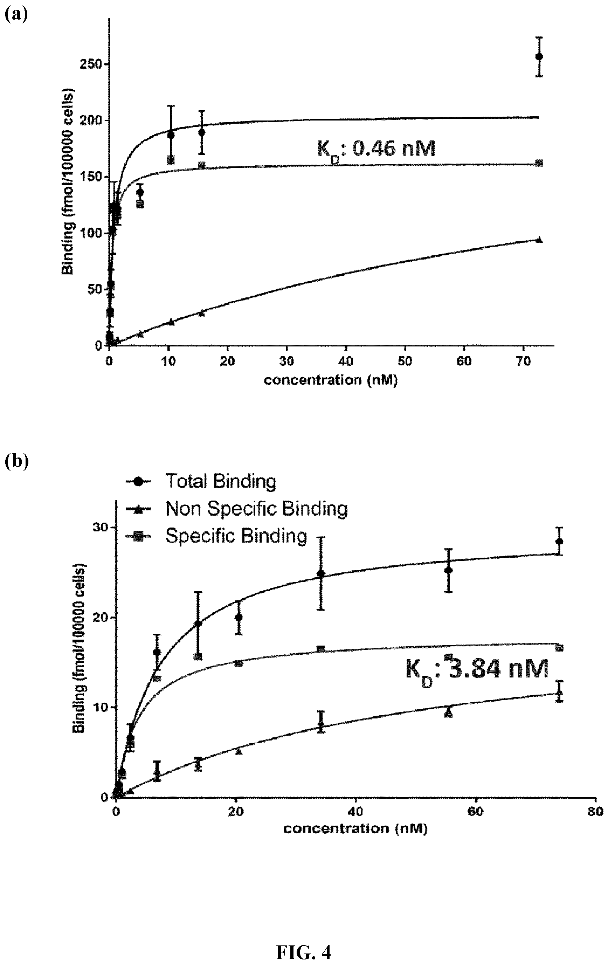

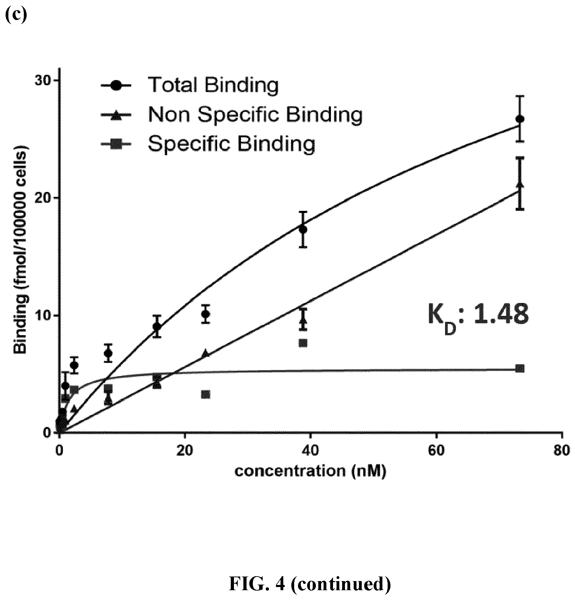

The saturation curve of radioligand binding analysis between the MC38-CD8 cells and the ¹²⁵I-CD8 single domain antibody is shown in FIG.4.

The measured binding affinity of the ¹²⁵I-CD8 single domain antibody is shown in Table 8. The binding of the ¹²⁵I labeled CD8 single domain antibody to the MC38-CD8 cells can be blocked by the unlabeled CD8 single domain antibody, which proves the specific binding ability of the ¹²⁵I labeled CD8 single domain antibody to the MC38-CD8 cells. The K_{D} value of ¹²⁵I-SNA006a is 0.46 nM, while the K_{D} value of ¹²⁵I-SNA006c is 3.8 nM and the K_{D} value of ¹²⁵I-SNA006d is 1.5 nM, wherein, the affinity of ¹²⁵I-SNA006a is significantly better than that of ¹²⁵I-SNA006-c and d. The results show that the CD8 single domain antibody has a significant affinity to a CD8 target protein, wherein ¹²⁵I-SNA006a has the strongest affinity.

**Table 8 Binding affinity of CD8 single domain antibody to CD8**

| | | |
|---|---|---|
| ¹²⁵I-SNA006a | K_{D} | 4.6 X 10⁻¹⁰ M |
| ¹²⁵I-SNA006c | K_{D} | 3.8 X 10⁻⁹ M |
| ¹²⁵I-SNA006d | K_{D} | 1.5 X 10⁻⁹ M |

### 4.4 Competition binding experiment of ¹²⁵I labeled CD8α single domain antibody (Competition binding assay)

Cell preparation: a saturation test was carried out in a 96-well filter plate. MC38-CD8 cells were cultured in a 50 mL culture dish. Before the test, a culture solution was removed, the cells were washed twice with 0.01 M sterile PBS, digested with trypsin and washed twice with PBS again, and finally prepared into a cell suspension with 5% BSA. The cells were added into the 96-well suction filter plate in a concentration of about 10⁵ cells/well.

The 96-well cell suction filter plate was saturated with a binding buffer and dried in air before use. About 10⁵ cells and the CD8 single domain antibody with gradually increased concentrations were added into each well of the suction filter plate, and then the ¹²⁵I-CD8 single domain antibody was added into each well at the rate of about 2×10⁵ counts/min, and the reaction volume was adjusted to 200 µL with the binding buffer

After reaction for 2 hours at 4°C, the reaction solution in the suction filter plate was drained using auxiliary equipment of a vacuum pump and the suction filter plate, 200 µL of PBS buffer (0.01 M, pH=7.4) was added into each well and drained, and the cells were repeatedly washed for 6 times. After the filter membrane at the bottom of the suction filter plate was dried, the filter membrane at the bottom was collected, and a γ counter was used for measurement. Calculation and plotting were performed by using Prism 7.0 (GraphPad Software, Inc.), and the half inhibition concentration IC50 was calculated by nonlinear regression analysis. Four parallel samples were set at each experimental point, and the experiment was repeated twice. The result given in this experiment is an average value plus a standard deviation.

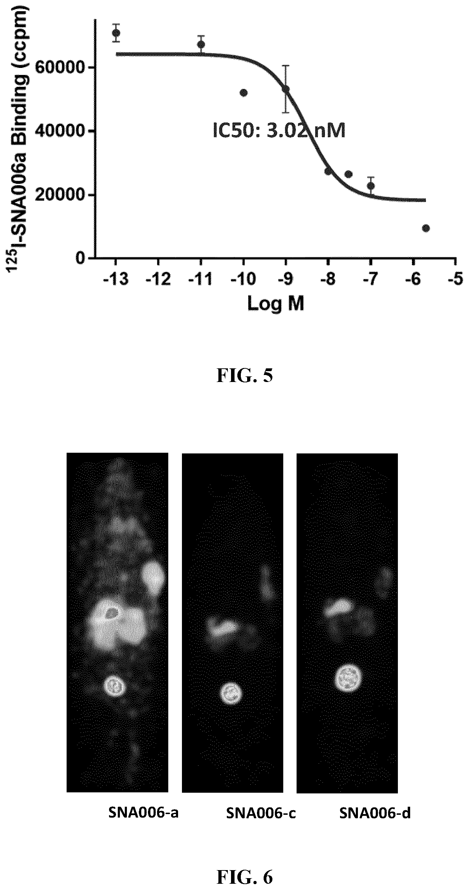

As shown in FIG. 5, the binding of ¹²⁵I-SNA006a to the MC38-CD8 cell receptor can also be inhibited by SNA006a "cold" protein in a concentration-dependent manner. The IC50 value obtained by Prism 7.0 (GraphPad Software, Inc.) software fitting is about 3.0 nM. The competition binding essay described herein shows the high affinity and high specificity of SNA006a to CD8 antigen.

### Example 5 ¹²⁵I labeled CD8α single domain antibody and its SPECT/CT imaging

### 5.1 Animal model for studying ¹²⁵I labeled CD8α single domain antibody

6-8 week old female nude mice were used for in-vivo study. The nude mice were fed in a specific pathogen free (SPF) environment, ate food and drunk at will, and were circularly lighten for standard 12-hour day and night. For xenotransplantation, 100 µL of cells (MC38-CD8 or MC38)/PBS was subcutaneously implanted at right front legs of nude mice. The inoculation density of cells was about 5-6 × 10⁶ cells/animal. Implantation was performed under isoflurane anesthesia. Under these conditions, in more than 90% of injected animals, suitable tumors (100-300 mm³) (MC38-CD8 or MC38) were obtained after 1-2 weeks.

After the experiment was ended, the tumor tissues were collected for CD8 immunohistochemical staining, and the expression level of CD8 was analyzed and compared with the overall CD8 expression level of the tumor detected by PET/CT.

### 5.2 In-vivo SPECT imaging with ¹²⁵I labeled CD8α single domain antibody

The MC38-CD8+/- tumor model was inoculated according to the method in 5.1. The nude mice were anaesthetized with isoflurane and then placed in an SPECT/CT bed, and administrated with about 10 µg of radiolabeled single domain antibody (about 100 µCi/10 µg) via tail vein. SPECT scanning was performed respectively in 1 h and 2h, and the radioactive absorption of tumor and tissue organs at multiple time points was analyzed. The results are shown in FIG. 6.

Within 120 min after the ¹²⁵I-CD8 single domain antibody was injected, the MC38-CD8 transplanted tumor was clearly visible. It can be seen from FIG. 6 that the right MC38-CD8+tumor has a good contrast compared with the contralateral negative control tumor MC38-CD8-, and the ¹²⁵I-CD8 single domain antibody has a significant concentration in kidney, which indicates that it is mainly metabolized by kidney. Because the above iodine labeling methods inevitably lead to deiodization, the SPECT results can lead to radioactive concentration in thyroid and stomach. Furthermore, ¹²⁵I energy is weak, and its application in SPECT imaging can cause certain tissue self-absorption. Therefore, the application of the ¹²⁵I labeled CD8 single domain antibody to SPECT imaging is intended to screen the best candidate antibody for use in the subsequent ⁶⁸Ga labeled imaging agent. The results show that ¹²⁵I labeled SNA006a, SNA006c and SNA006d have high uptake at tumor sites, and the imaging effect of SNA006a is significantly better than the imaging effects of SNA006c and SNA006d. The in-vivo screening experimental results of SPECT are consistent with corresponding in-vitro experimental results, which further proves that SNA006a has the strongest CD8 tracing potential among all the aforementioned single domain antibodies.

### Example 6 Synthesis of NOTA-CD8α single domain antibody precursor and radiolabeling of ⁶⁸Ga-NOTA-CD8α single domain antibody

### 6.1 Synthesis of NOTA-CD8α single domain antibody precursor

The bifunctional chelating agent p-SCN-NOTA was coupled to the CD8 single domain antibody. The coupling product was purified via a PD-10 column and the buffer was replaced to normal saline. The absorbance at 280nm was measured by an ultraviolet spectrophotometer, and the coupling efficiency was analyzed by ESI-Q-TOF-MS. The targeting of the coupling product to tumor cells was studied by using an in-vitro cell test (tumor cell binding and affinity detection), and the in-vitro affinity to a CD8α protein was analyzed by ELISA.

By using PD-10 column (GE), the buffer of the CD8α-cHis single domain antibody (including C37, C46 and S5) was changed to a NaHCO₃ buffer solution with a certain pH, and p-SCN-Bn-NOTA (Macrocyclics, article No. B-605) was dissolved into DMSO with a concentration of 25 mg/mL, and the p-SCN-Bn-NOTA solution whose molar was twice that of lysine on the single domain antibody was added into the CD8α single domain antibody, the above materials reacted for a certain time period at room temperature, the conjugated product was purified by PD-10 column (GE) and concentrated by ultrafiltration.

The activity of the above CD8α-NOTA after labeling was determined by ELISA. The specific method is as follows: the CD8α-Fc fusion protein was placed in the coated plate in a concentration of 5 µg/well at 4°C overnight. After blocking with 3% BSA at 37°C, samples subjected to gradient dilution (the uncoupled CD8α single domain antibody was a standard substance) were added to act for 1 hour at 37°C. Then, anti-his-HRP (purchased from Abeam Company) was added to react for 1 hour at room temperature. Then, a chromogenic solution was added, and the absorption value at 450 nm was read. The activities of ELISA are summarized in Table 9.

**Table 9 ELISA activity result summary of CD8α-cHis-NOTA**

| Single domain antibody | C37 | C46 | S5 |
|---|---|---|---|
| Relative activity of CD8α-cHis-NaHCO₃ (%) | 76 | 76 | 98 |
| Relative activity of CD8α-cHis-NOTA (%) | 77 | 20 | 105 |

### 6.2 Radiolabelling of ⁶⁸Ga-NOTA-CD8α single domain antibody

⁶⁸Ga leacheate was prepared by leaching an Eckert&Ziegler IGG100 germanium 68/gallium 68 (Ge 68/Ga 68) generator with 0.1 M sterile HCl, an equal volume of 0.2 M sodium acetate solution was added, and a 1/4 volume of 0.1 M sodium acetate buffer containing the above NOTA-CD8 antibody and having the pH of 5.3 was added, the pH of the reaction system was between 4.5 and 4.7, and the above materials reacted for 10 minutes at room temperature. The unreacted ion gallium was removed via PD-10 column while exchanging the product buffer system to normal saline, and the product was filtered with a 0.22 µm filter membrane. The quality of the product was controlled by analyzing pH, Radio-TLC, Radio-HPLC, radioactivity, radiochemical purity and the like. In addition, the ⁶⁸Ga leacheate was also prepared by leaching an ITG germanium 68/gallium 68 (Ge-68/Ga-68) generator with 0.05 M sterile HCl, a 1/2 volume of 0.2 M sodium acetate solution was added, and other reaction and quality control conditions were the same.

### 6.3 Instant thin-layer chromatography (ITLC) analysis

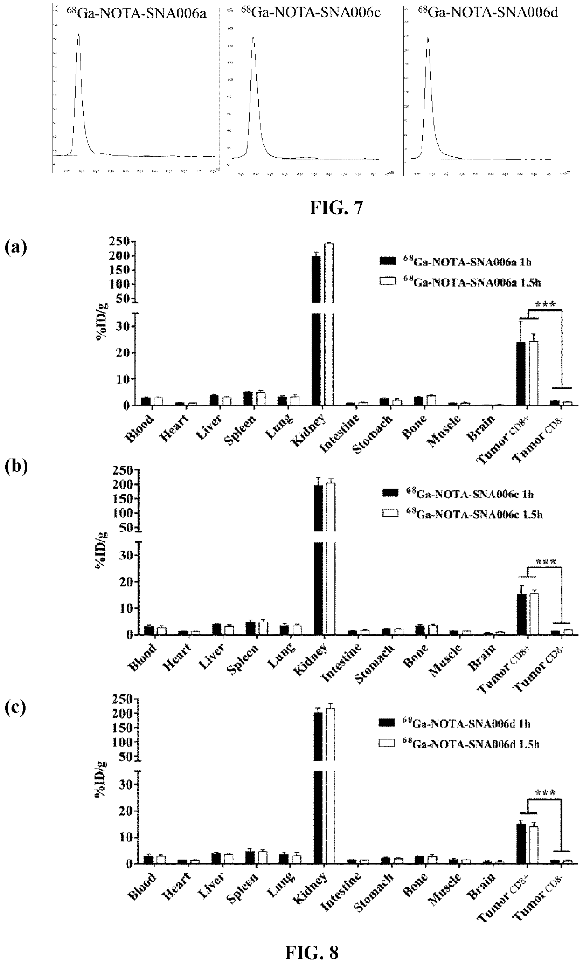

ITLC-SG were pre-cut into 1 cm × 12 cm strips and a distance between ends of strips was labeled with a pencil. A developing solvent was poured into a developing chamber until the tank was covered and the developing solvent was balanced. A drop of ⁶⁸Ga-CD8 single domain antibody injection was dropwise added at a pencil line that was distanced from the bottom of the ITLC strip by 1 cm. The ITLC strip was placed in the developing chamber and developed at a position distanced from a sampling point by 10 cm (i.e., to a top pencil line mark). LTIC was scanned with a radiometric ITLC scanner, and the radiochemical purity (RCP) was calculated by integrating the peaks on a chromatogram. The analysis results are shown in FIG. 7, indicating that the CD8 single domain antibody had successfully been labeled with the positron nuclide ⁶⁸Ga .

### Example 7 In-vivo distribution of ⁶⁸Ga-NOTA-CD8α single domain antibody

### 7.1 Animal model for studying ⁶⁸Ga-NOTA-CD8α single domain antibody

6-8 week old female nude mice were used for in-vivo study. The nude mice were fed in a specific pathogen free (SPF) environment, ate food and drunk at will, and were circularly lighten for standard 12-hour day and night. For xenotransplantation, 100 µL of cells (MC38-CD8 or MC38)/PBS was subcutaneously implanted at right front legs of the nude mice. The inoculation density of cells was about 5-6 × 10⁶ cells/animal. Implantation was performed under isoflurane anesthesia. Under these conditions, in more than 90% of injected animals, suitable tumors (100-300 mm³) (MC38-CD8 or MC38) were obtained after 1-2 weeks.

After the experiment was ended, the tumor tissues were collected for CD8 immunohistochemical staining, and the expression level of CD8 was analyzed and compared with the overall CD8 expression level of the tumor detected by PET/CT.

### 7.2 In-vivo distribution of ⁶⁸Ga-NOTA-CD8α single domain antibody

The MC38-CD8+/- tumor model was inoculated according to the method in 7.1, and about 10 µg of radiolabeled single domain antibody (about 100 µCi/10 µg) was administrated to nude mice via tail vein. Data were collected at 1 and 1.5 hours, and 3 nude mice were euthanized at each time point. Target tissues such as blood, kidney, liver, spleen, lung, heart, intestine, stomach, muscle, skin, brain, bone and CD8+/- tumor were dissected, and counting was performed on the γ counter to collect data. The injection dose was used as a total injection dose. For each organ, the % injection dose (% ID) was determined based on the total injection dose, and the organs were weighed to determine the % injection dose per gram (% ID/g).

FIG. 8 shows biological distribution data of ⁶⁸Ga-NOTA-CD8α single domain antibody in-vivo ingestion in intact male mice with MC38-CD8 tumors, which is ex-vivo biological distribution data of ⁶⁸Ga-NOTA-CD8 ingestion in intact male mice with subcutaneous tumors (n=3). Data were collected 1 and 1.5 hours after intravenous administration. The data is expressed in mean% ID/g ± standard deviation (S.D.). The error of the ratio is calculated by the geometric mean of the standard deviation. As shown in FIG. 8, the error bar represents the standard deviation of this group.

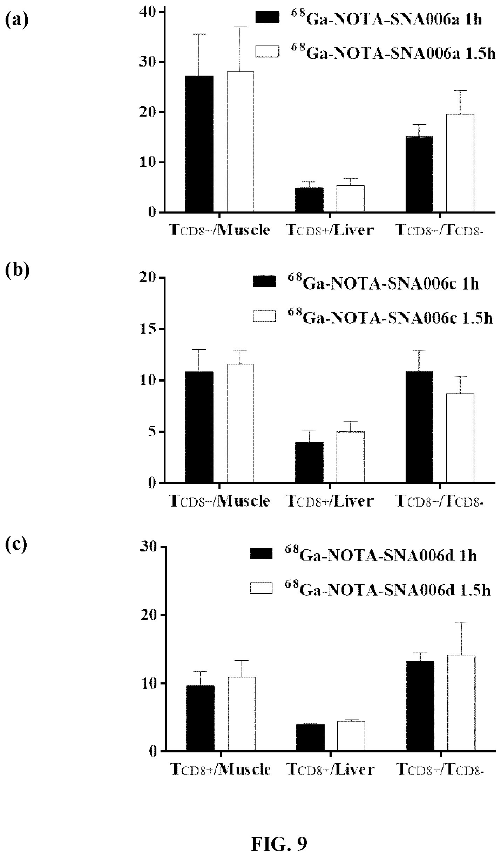

FIG. 9 shows results of tumor tissue and blood ingestion ratios and tumor tissue and contralateral normal muscle ingestion ratios in these experiments. The CD8 single domain antibody shows good tumor ingestion in MC38-CD8 tumors expressing the target. The peak tumor/muscle ratio is high, which shows that ⁶⁸Ga-NOTA-SNA006a single domain antibody has good tumor specificity. The label ⁶⁸Ga-NOTA-SNA006a may become a good tumor imaging agent.

### Example 8 In-vivo PET imaging with ⁶⁸Ga-NOTA-CD8α single domain antibody

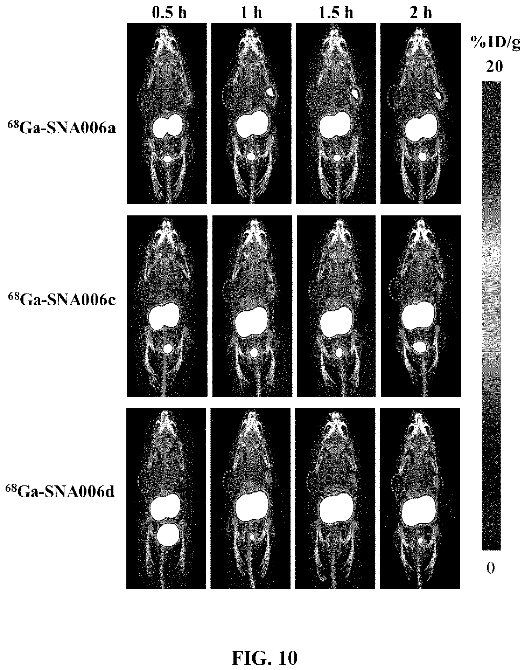

The MC38-CD8+/- tumor model was inoculated according to the method in 6.1. Micro-PET/CT (IRIS PET/CT, Inviscan, Strasbourg, France) scanning was used to measure focus % ID/g. continuous PET/CT scanning of small animals was performed for 120 minutes to analyze the radioactive absorption of tumor, muscle and kidney at multiple time points, as shown in FIG.10. The Monte-Carlo based 3D OSEM algorithm was used for image reconstruction. The radioactivity (MBq/mL) was calculated by a region of interest (ROI) method in tumor, muscle, liver and other organs. The obtained value was divided by injection dose to obtain the ingestion value of each tissue on PET tracer (% ID/g) (assuming that the tissue density was 1 g/ml). The calculation results are shown in FIG.10.

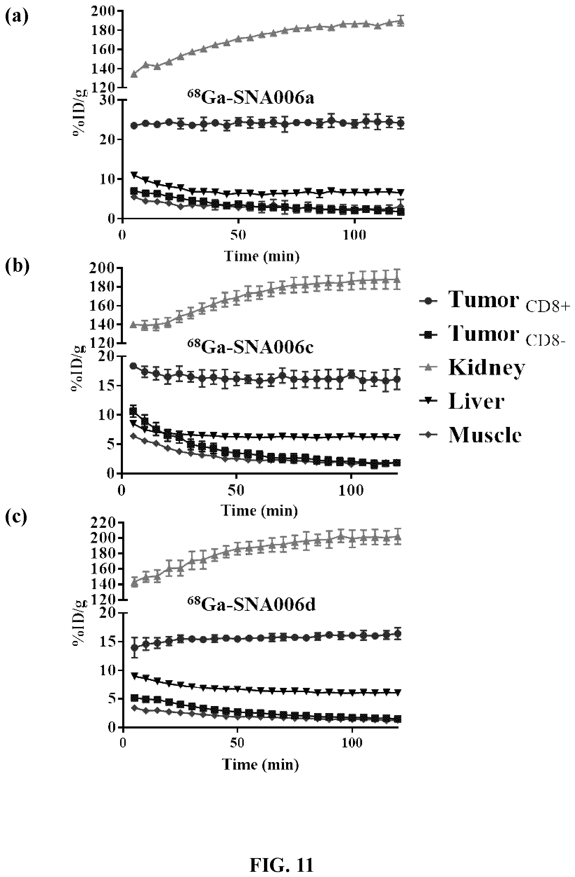

The results in FIG.10 show that 120 min after the above PET tracer ⁶⁸Ga-NOTA-SNA006a was injected, the MC38-CD8 transplanted tumor was clearly visible, while the MC38 transplanted tumor with CD8 negative expression was not ingested after the injection. Therefore, the MC38-CD8 tumor has good discrimination degree compared with a contralateral negative control tumor. According to PET scanning results, the radioactivity absorption of tumors, muscles and kidneys at multiple time points was analyzed. The ROI results of biological distribution were analyzed by using an activity time curve, as shown in FIG.11.

The results show that all of ⁶⁸Ga-NOTA-SNA006a, ⁶⁸Ga-NOTA-SNA006c and ⁶⁸Ga-NOTA-SNA006d have higher ingestion at tumor sites, and the imaging effect of ⁶⁸Ga-NOTA-SNA006a is significantly superior to that of c and d. The in-vivo screening experimental results of PET are consistent with corresponding in-vitro experimental results, which further proves that ⁶⁸Ga-NOTA-SNA006a has the strongest CD8 tracing potential among all the above single domain antibodies.

### Example 9 In-vivo stability experiment of ⁶⁸Ga-NOTA-CD8α single domain antibody

Normal mice were taken and ⁶⁸Ga-NOTA-SNA006a 74 MBq (1 mCi/100µL PBS) from tail vein. After 0.5 h and 1 h of injection, urine samples were collected, all samples were dissolved with a certain amount of 50% acetonitrile aqueous solution and then centrifuged for 15 min at 8000 rpm, and the supernatant was taken to pass through a 0.22 µm filter membrane, subsequently, the filtrate was analyzed with Radio-TLC.

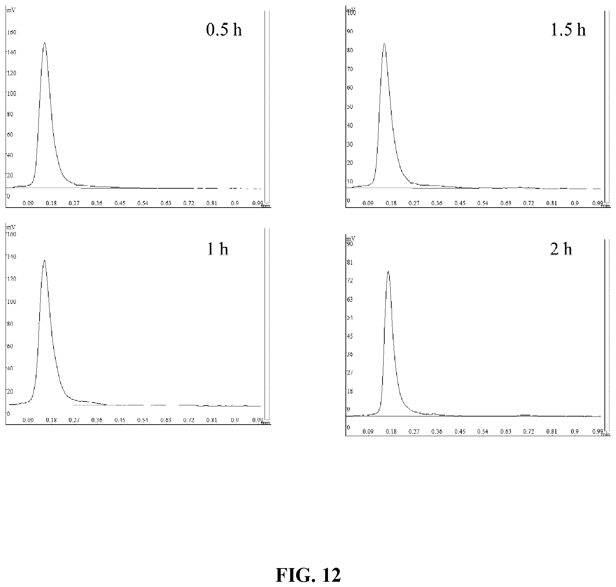

Radio-TLC results are shown in FIG.12. In the figure, there is no significant degradation or ⁶⁸Ga shedding phenomenon, indicating that under this condition, the radiolabeled antibody is stable in structure and good in in-vivo stability.

## Claims

1. A CD8 binding polypeptide, comprising at least one immunoglobulin single variable domain capable of specifically binding to CD8α, the at least one immunoglobulin single variable domain comprises CDR1, CDR2 and CDR3 of any one of SEQ ID NOs: 5, 9, 13, 17, 21, 25, 29, 33, 37, 41, 45, 49, 53, 57, 61, 65, 69, 73, 77 and 81.

2. The CD8 binding polypeptide according to claim 1, wherein the at least one immunoglobulin single variable domain comprises the CDR1, CDR2 and CDR3 selected from:
(1) CDR1 of SEQ ID NO:2, CDR2 of SEQ ID NO: 3 and CDR3 of SEQ ID NO:4;
(2) CDR1 of SEQ ID NO:6, CDR2 of SEQ ID NO: 7 and CDR3 of SEQ ID NO:8;
(3) CDR1 of SEQ ID NO: 10, CDR2 of SEQ ID NO: 11 and CDR3 of SEQ ID NO:12;
(4) CDR1 of SEQ ID NO:14, CDR2 of SEQ ID NO: 15 and CDR3 of SEQ ID NO:16;
(5) CDR1 of SEQ ID NO:18, CDR2 of SEQ ID NO: 19 and CDR3 of SEQ ID NO:20;
(6) CDR1 of SEQ ID NO:22, CDR2 of SEQ ID NO: 23 and CDR3 of SEQ ID NO:24;
(7) CDR1 of SEQ ID NO:26, CDR2 of SEQ ID NO: 27 and CDR3 of SEQ ID NO:28;
(8) CDR1 of SEQ ID NO:30, CDR2 of SEQ ID NO: 31 and CDR3 of SEQ ID NO:32;
(9) CDR1 of SEQ ID NO:34, CDR2 of SEQ ID NO: 35 and CDR3 of SEQ ID NO:37;
(10) CDR1 of SEQ ID NO:38, CDR2 of SEQ ID NO: 39 and CDR3 of SEQ ID NO:40;
(11) CDR1 of SEQ ID NO:42, CDR2 of SEQ ID NO: 43 and CDR3 of SEQ ID NO:44;
(12) CDR1 of SEQ ID NO:46, CDR2 of SEQ ID NO: 47 and CDR3 of SEQ ID NO:48;
(13) CDR1 of SEQ ID NO:50, CDR2 of SEQ ID NO: 51 and CDR3 of SEQ ID NO:52;
(14) CDR1 of SEQ ID NO:54, CDR2 of SEQ ID NO: 55 and CDR3 of SEQ ID NO:56;
(15) CDR1 of SEQ ID NO:58, CDR2 of SEQ ID NO: 59 and CDR3 of SEQ ID NO:60;
(16) CDR1 of SEQ ID NO:62, CDR2 of SEQ ID NO: 63 and CDR3 of SEQ ID NO:64;
(17) CDR1 of SEQ ID NO:66, CDR2 of SEQ ID NO: 67 and CDR3 of SEQ ID NO:68;
(18) CDR1 of SEQ ID NO:70, CDR2 of SEQ ID NO: 71 and CDR3 of SEQ ID NO:72;
(19) CDR1 of SEQ ID NO:74, CDR2 of SEQ ID NO: 75 and CDR3 of SEQ ID NO:76;
(20) CDR1 of SEQ ID NO:78, CDR2 of SEQ ID NO: 79 and CDR3 of SEQ ID NO:80.

3. The CD8 binding polypeptide according to claim 1 or 2, wherein the immunoglobulin single variable domain comprises an amino acid sequence having at least 80%, preferably at least 90%, more preferably at least 95%, even more preferably at least 99% sequence identity to an amino acid sequence of one of SEQ ID NOs: 5, 9, 13, 17, 21, 25, 29, 33, 37, 41, 45, 49, 53, 57, 61, 65, 69, 73, 77 and 81.

4. The CD8 binding polypeptide according to any one of claims 1-3, wherein the immunoglobulin single variable domain comprises an amino acid sequence of one of SEQ ID NOs: 5, 9, 13, 17, 21, 25, 29, 33, 37, 41, 45, 49, 53, 57, 61, 65, 69, 73, 77 and 81.

5. The CD8 binding polypeptide according to any one of claims 1-4, wherein the immunoglobulin single variable domain is a VHH.

6. A nucleic acid molecule encoding the CD8 binding polypeptide according to any one of claims 1-5.

7. An expression vector, comprising the nucleic acid molecule according to claim 6 operatively linked to an expression regulatory element.

8. A host cell, comprising the nucleic acid molecule according to claim 6 or being transformed by the expression vector according to claim 7, and being capable of expressing the CD8 binding polypeptide.

9. A method for producing the CD8 binding polypeptide according to any one of claims 1-5, comprising:
a) culturing the host cell according to claim 8 under the condition allowing the expression of the CD8 binding polypeptide;
b) recovering the CD8 binding polypeptide expressed by the host cell from the culture obtained in step a); and
c) optionally further purifying and/or modifying the CD8 binding polypeptide obtained in step b).

10. A conjugated molecule, comprising the CD8 binding polypeptide according to any one of claims 1-5, and at least one detectable label conjugated to the CD8 binding polypeptide.

11. The conjugated molecule according to claim 10, wherein the detectable label is selected from a radionuclide, a fluorescent agent, a chemiluminescent agent, a bioluminescent agent, a paramagnetic ion and an enzyme.

12. The conjugated molecule according to claim 11, wherein the detectable label is selected from ¹¹⁰In, ¹¹¹In, ¹⁷⁷Lu, ¹⁸F, ⁵²Fe, ⁶²Cu, ⁶⁴Cu, ⁶⁷Cu, ⁶⁷Ga, ⁶⁸Ga, ⁶⁸Ge, ⁸⁶Y, ⁹⁰Y, ⁸⁹Zr, ^{94m}Tc, ¹²⁰I, ¹²³I, ¹²⁴I, ¹²⁵I, ¹³¹I, ¹⁵⁴⁻¹⁵⁸Gd, ³²P, ¹¹C, ¹³N, ¹⁵O, ¹⁸⁶Re, ¹⁸⁸Re, ⁵¹Mn, ^{52m}Mn, ⁵⁵Co, ⁷²As, ⁷⁵Br, ⁷⁶Br, ⁸²mRb, ⁸³Sr or other γ-, β- or positive emitters, for example the detectable label is ⁶⁸Ga or ¹²⁵I.

13. The conjugated molecule according to any one of claims 10-12, wherein the CD8 binding polypeptide is conjugated with the detectable label through a chelating agent.

14. The conjugated molecule according to claim 13, wherein the chelating agent is selected from DTPA, EDTA, NOTA, DOTA, TRAP, TETA, NETA, CB-TE2A, Cyclen, Cyclam, Bispidine, TACN, ATSM, SarAr, AmBaSar, MAG₃, MAG₂, HYNIC, DADT, EC, NS₃, H2dedpa, HBED, DFO, PEPA or HERA, and derivatives thereof.

15. The conjugated molecule according to claim 14, wherein the detectable label is ⁶⁸Ga and the chelating agent is NOTA.

16. A method for detecting the presence and/or amount of CD8 in a biological sample, comprising:
a) contacting the biological sample and a control sample with the CD8 binding polypeptide of any one of claims 1-5 or the conjugated molecule of any one of claims 10-15 under the condition that the CD8 binding polypeptide according to any one of claims 1-5 or the conjugated molecule according to any one of claims 10-15 can form a complex with CD8; and
b) detecting the formation of the complex,
wherein, the difference in the formation of the complex between the biological sample and the control sample indicates the presence and/or amount of CD8 in the sample.

17. A detecting agent for detecting a CD8 positive cell, comprising the CD8 binding polypeptide according to any one of claims 1-5 or the conjugated molecule according to any one of claims 10-15, and optionally a physiologically acceptable carrier.

18. The detecting agent according to claim 17, wherein the detecting agent is a contrast agent.

19. The detecting agent according to claim 18, wherein the contrast agent is an Emission Computed Tomography (ECT) contrast agent, such as a Single photon Emission Computed Tomography (SPECT) contrast agent or a Positron Emission Tomography (PET) contrast agent.

20. Use of the CD8 binding polypeptide according to any one of claims 1-5 or the conjugated molecule according to any one of claims 10-15 in preparation of a detecting agent for detecting a CD8 positive cell.

21. The detecting agent according to claim 20, wherein the detecting agent is a contrast agent.

22. The detecting agent according to claim 21, wherein the contrast agent is an ECT contrast agent, such as an SPECT contrast agent or a PET contrast agent.

23. A method for detecting the presence and/or amount of CD8 positive cell(s) in a tissue, comprising:
a) contacting the tissue with the conjugated molecule according to any one of claims 10-15 or the detecting agent according to any one of claims 17-19; and
b) determining the presence and/or amount of the CD8 positive cell(s) in the tissue.

24. The method according to claim 23, wherein the tissue is selected from a blood tissue, a lymphatic tissue and a tumor tissue.

25. The method according to claim 23 or 24, wherein the CD8 positive cell(s) is/are CD8 positive T cell(s).

26. The method according to any one of claims 23-25, wherein the presence and/or amount of the CD8 positive cell(s) in the tissue is determined by imaging the tissue.

27. The method according to any one of claims 23-25, wherein the presence and/or amount of the CD8 positive cell(s) in the tissue is determined by flow cytometry.

28. A method for detecting the presence and/or amount of CD8 positive cell(s) in a tissue of a subject, comprising administrating the conjugated molecule according to any one of claims 10-15 or the detecting agent according to any one of claims 17-19 to the subject.

29. The method according to claim 28, wherein the tissue is a tumor tissue.

30. The method according to claim 28 or 29, wherein the CD8 positive cell(s) is/are CD8 positive T cell(s).

31. The method according to any one of claims 28-30, wherein the method further comprises a step of imaging the subject, such as by ECT imaging, for example the ECT imaging is SPECT imaging or PET imaging.

32. A method for determining whether a subject with a tumor is suitable for an anti-tumor therapy, the method comprising:
1) administrating the conjugated molecule according to any one of claims 10-15 or the detecting agent according to any one of claims 17-19 to the subject, and
2) imaging the subject, such as by ECT imaging, to determine whether the tumor of the subject contains CD8 positive cell(s),
wherein, if the presence of CD8 positive cell(s) in the tumor is detected, for example the tumor of the subject is infiltrated by CD8 positive cell(s), the subject is identified as being suitable for the anti-tumor therapy.

33. A method for predicting the response of a subject with a tumor to an anti-tumor therapy, the method comprising:
1) administrating the conjugated molecule according to any one of claims 10-15 or the detecting agent according to any one of claims 17-19 to the subject, and
2) imaging the subject, such as ECT imaging, to determine whether the tumor of the subject contains CD8 positive cell(s),
wherein, if the presence of CD8 positive cell(s) in the tumor is detected, for example the tumor of the subject is infiltrated by CD8 positive cell(s), the subject may respond to the anti-tumor therapy.

34. A method for treating a tumor in a subject, the method comprising:
1) administrating the conjugated molecule according to any one of claims 10-15 or the detecting agent according to any one of claims 17-19 to the subject, and
2) imaging the subject, such as ECT imaging, to determine whether the tumor of the subject contains the CD8 positive cell(s),
wherein, if the presence of CD8 positive cell(s) in the tumor is detected, for example the tumor of the subject is infiltrated by CD8 positive cell(s), applying the anti-tumor therapy to the subject.

35. A method for monitoring the efficacy of an anti-tumor therapy in a subject, the method comprising:
1) administrating the conjugated molecule according to any one of claims 10-15 or the detecting agent according to any one of claims 17-19 to a subject with a tumor and treated with the anti-tumor therapy; and
2) imaging the subject, such as by ECT imaging, to determine the amount of the CD8 positive cell(s) in the tumor of the subject.

36. The method according to any one of claims 32-35, wherein the anti-tumor therapy is an immunocheckpoint inhibitor therapy.

37. The method according to any one of claims 32-36, wherein the anti-tumor therapy is selected from administration of PD-1 inhibitors, PD-L1 inhibitors, CTLA-4 inhibitors, TIM3 inhibitors, BTLA inhibitors, TIGIT inhibitors, CD47 inhibitors, GITR inhibitors, LAG3 inhibitors, antagonists of another T cell co-inhibitors or ligand, indoleamine-2,3-dioxygenase (IDO) inhibitors, vascular endothelial growth factor (VEGF) antagonists, Ang2 inhibitors, transforming growth factor β (TGFβ) inhibitors, epidermal growth factor receptor (EGFR) inhibitors, CD20 inhibitors, antibodies for tumor-specific antigens, vaccines, adjuvants to increase antigen presentation, bispecific antibodies, cytotoxics, chemotherapeutic agents, cyclophosphamide, radiotherapy, IL-6R inhibitors, IL-4R inhibitors, IL-10 inhibitors, cytokines and antibody-drug conjugates (ADC).

38. The method according to any one of claims 32-37, wherein the tumor is a solid tumor.

39. The method according to claim 38, wherein the solid tumor is selected from colorectal cancer, ovarian cancer, prostate cancer, breast cancer, brain cancer, cervical cancer, bladder cancer, anal cancer, uterine cancer, colon cancer, liver cancer, pancreatic cancer, lung cancer, endometrial cancer, bone cancer, testicular cancer, skin cancer, kidney cancer, stomach cancer, esophageal cancer, head and neck cancer, salivary gland cancer, and myeloma.

40. A method for isolating and/or purifying CD8 positive cell(s), the method comprising:
(a) providing a cell population suspected to contain CD8 positive cell(s);
(b) identifying a subpopulation of the cell population, wherein the cells of the subpopulation bind the CD8 binding polypeptide according to any one of claims 1-5 or the conjugated molecule according to any one of claims 10-15; and
(c) isolating the subpopulation.

41. A method for isolating CD8 positive cell(s), the method comprising:
(a) providing a cell population suspected to contain CD8 positive cell(s);
(b) contacting the cell population with the CD8 binding polypeptide according to any one of claims 1-5 or the conjugated molecule according to any one of claims 10-15, thereby allowing the CD8 positive cell(s) to bind to the CD8 binding polypeptide according to any one of claims 1-5 or the conjugated molecule according to any one of claims 10-15;
(c) removing cell(s) that do not bind to the CD8 binding polypeptide according to any one of claims 1-5 or the conjugated molecule according to any one of claims 10-15; and
(d) recovering the CD8 positive cell(s) that bind to the CD8 binding polypeptide according to any one of claims 1-5 or the conjugated molecule according to any one of claims 10-15.

42. The method according to claim 40 or 41, wherein the CD8 positive cell(s) is/are CD8 positive T cell(s).

43. The method according to any one of claims 40-42, wherein the cell population comprising the CD8 positive cell(s) is human peripheral blood mononuclear cell (PBMC).

44. The method according to any one of claims 40-44, wherein the CD8 binding polypeptide according to any one of claims 1-5 or the conjugated molecule according to any one of claims 10-15 is immobilized onto a solid surface, for example is immobilized onto a gel or the surface of a magnetic bead.

45. A kit, comprising the CD8 binding polypeptide according to any one of claims 1-5 or the conjugated molecule according to any one of claims 10-15 or the detecting agent according to any one of claims 17-19.
